(19) Europäisches Patentamt
European Patent Office
Office européen des brevets

(11) EP 3 107 520 B1

(12) # EUROPÄISCHE PATENTSCHRIFT

(45) Veröffentlichungstag und Bekanntmachung des Hinweises auf die Patenterteilung:
**30.10.2019 Patentblatt 2019/44**

(21) Anmeldenummer: **15704566.7**

(22) Anmeldetag: **17.02.2015**

(51) Int Cl.:
***A61K 6/00*** (2006.01) ***A61K 6/083*** (2006.01)

(86) Internationale Anmeldenummer:
**PCT/EP2015/053303**

(87) Internationale Veröffentlichungsnummer:
**WO 2015/124559 (27.08.2015 Gazette 2015/34)**

(54) **POLYMERISIERBARES DENTALMATERIAL**

POLYMERIZABLE DENTAL MATERIAL

MATÉRIAU DENTAIRE POLYMÉRISABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.02.2014 DE 102014203166**

(43) Veröffentlichungstag der Anmeldung:
**28.12.2016 Patentblatt 2016/52**

(73) Patentinhaber: **Mühlbauer Technology GmbH**
**22547 Hamburg (DE)**

(72) Erfinder:
- **BÖTTCHER, Henrik**
  **21255 Tostedt (DE)**
- **NEFFGEN, Stephan**
  **25421 Pinneberg (DE)**

(74) Vertreter: **Glawe, Delfs, Moll**
**Partnerschaft mbB von**
**Patent- und Rechtsanwälten**
**Postfach 13 03 91**
**20103 Hamburg (DE)**

(56) Entgegenhaltungen:

| | |
|---|---|
| **EP-A1- 2 226 061** | **EP-A2- 2 153 811** |
| **WO-A1-96/33157** | **DE-A1- 19 848 886** |
| **US-A- 6 084 004** | **US-A1- 2006 052 470** |
| **US-A1- 2011 275 035** | **US-B2- 6 620 864** |

- **N.S. Allen et al.: "Photochemistry and photo-induced co-synergistic polymerisation activities of novel N,N,-dimethylaminobenzoates and benzamides", , 4. Dezember 2000 (2000-12-04), XP055186056, Gefunden im Internet: URL:http://www.sciencedirect.com/science/article/pii/S1010603000003646 [gefunden am 2015-04-27]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 107 520 B1

## Beschreibung

Die Erfindung betrifft ein polymerisierbares Dentalmaterial gemäß dem Oberbegriff des Anspruchs 1. Gegenstand der Erfindung ist ferner die Verwendung eines tertiären aromatisches Amins gemäß Anspruch 13 als Coinitiator in einem polymerisierbaren Dentalmaterial. Gegenstand der Erfindung ist auch ein Coinitiator mit verbesserter Wasserlöslichkeit sowie ein radikalisch polymerisierbares dentales Restaurationsmaterial enthaltend den Coinitiator, dessen Haftkraft am Zahn, bei einfachster Anwendung, verbessert wird. Das radikalisch polymerisierbare Dentalmaterial ist bevorzugt ein fließfähiges, selbstätzendes, selbsthaftendes Komposit für direkte Füllungen.

An dentale Restaurationsmaterialien werden besondere Anforderungen gestellt. Dies gilt insbesondere für direkte Füllungsmaterialien, Befestigungszemente oder Adhäsive, die eine polymerisierbare Säure als adhäsive und/oder ätzende Komponente enthalten, intraoral appliziert und lichtgehärtet werden.

Die dentalen Restaurationsmaterialien müssen biologisch und toxikologisch unbedenklich sein, eine möglichst hohe Durchhärtetiefe (DHT), einen geringen Polymerisationsschrumpf, eine hohe Lagerstabilität (2 oder mehr Jahre, bevorzugt bei Raumtemperatur) und geringe Empfindlichkeit gegenüber Umgebungslicht sowie im ausgehärteten Zustand die gewünschte Lichtopazität, Farbstabilität, Röntgenopazität, eine geringe Wasseraufnahme (WA) und Wasserlöslichkeit (WS) sowie eine möglichst hohe Biegefestigkeit (BF) aufweisen. Die polymerisierte Restauration soll insbesondere eine möglichst hohe Scherhaftkraft (SBS) auf Dentin aufweisen.

Insbesondere um die SBS von Restaurationsmaterialien auf Dentin zu steigern, ist es bekannt, dentalen Restaurationsmaterialien höhere Anteile an polymerisierbaren Säuren zuzufügen.

Bevorzugt ist auch ein Anteil hydrophiler Monomere (Wasserlöslichkeit >50 g/l) enthalten, die keine Säuregruppe enthalten. Bevorzugt sind solche hydrophile Monomere, die in jedem Verhältnis mit Wasser mischbar sind, zu nennen wäre hier insbesondere 2-Hydroxethylmethacrylat (HEMA).

Der Polymerisationsschrumpf und die Festigkeit dentaler Restaurationsmaterialien kann durch Zugabe von bekannten Vernetzern und anorganischen Füllstoffen verbessert werden. Als Vernetzer werden bevorzugt Mischungen von Di(meth)acrylat auf Basis von Bisphenol-A und einem niedermolekularen Vernetzer wie Triethylenglycoldimethacrylat (TEDMA) eingesetzt. Geeignete anorganische Füllstoffe weisen Teilchengrößen unter 30 $\mu$m und eine hydrophobisierte Oberflächene auf. Als anorganische Füllstoffe werden bevorzugt röntgenopake silanisierte Gläser eingesetzt.

Bekannte selbsthaftende und selbstätzende radikalisch polymerisierbare dentale Restaurationsmaterialien, insbesondere fließfähige Komposite für die direkte Füllung, weisen Verbesserungsbedarf bezüglich oben genannter Eigenschaften auf. Insbesondere besteht weiterhin Bedarf die SBS fließfähiger Komposite an Dentin zu verbessern, ohne dass dieses zuvor mit separaten Ätzmitteln und/oder separaten Haftvermittlern vorbereitet wurde.

Bekannte lichthärtende selbsthaftende und selbstätzende radikalisch polymerisierbare Dentalrestaurative enthalten einen Photoinitiator und ein tertiäres aromatisches Amin als Coinitiator. Besonders bevorzugt wird eine Kombination aus Campherchinon und/oder Phosphinoxid-Verbindungen als Photoinitiatoren sowie Alkyl-(4-Dialkylaminobenzoesäure)-ester oder Butoxyethyl-(4-Dialkylaminobenzoesäure)-ester als Coinitiatoren. Solche Materialien sind vielfach beschrieben, beispielhaft seien hier folgende Dokumente genannt WO2011139936, EP2286784, EP2153811, WO2007100569, EP1784155, EP1387657 und EP0136186.

In Bezug auf vorgenanntes Dokument WO2011139936 bzw. dessen Patentfamilienmitglied US 2011/0275035 A1 sei angemerkt, dass das in diesen Dokumenten offenbarte Dentalmaterial kein mindestens eines tertiäres aromatisches Amin als Coinitiator aufweist, das mindestens eine weitere Gruppe, ausgewählt aus i. Carbonsäureestergruppen enthaltend mindestens eine Polyoxyalkylengruppe mit mindestens 2 Oxyethylen- und/oder Oxypropyleneinheiten und ii. Amidgruppe, direkt an einem Benzolring aufweist, wodurch eine verbesserte Löslichkeit und damit einhergehende erhöhte Amphiphilie des Coinitiators resultiert. Stattdessen werden lediglich die Verbindungen 4-Dimethylaminobenzoesäure-ethylester, 4-Dimethylaminobenzoesäure-2-ethylhexylester und 4-Dimethylaminobenzonitril als Coinitiatoren verwendet.

In der WO 00/49064 bzw. dessen Patentfamilienmitglied US 6 620 846 B2 wird ein kationisch härtbares dentales Restaurativ beschrieben, welches $\omega$-Ethylpoly(oxyethylen)-4-[bis($\omega$-(2-hydroxy-ethyl) poly(oxyethylen))-amino]benzoat (PEG-25-PBA) als Coinitiator enthalten soll. Dieser Coinitiator wird dort jedoch als den Alkyl-(4-Dialkylaminobenzoesäure)-estern gegenüber nachteilig beschrieben, da er eine nur geringe Reaktivität aufweist. Im Unterschied zu vorliegender Erfindung wird diesen Dokumenten auch kein Coinitiator umfassend einen Benzolring mit mindestens einer Dialkylamingruppe offenbart, sondern lediglich ein mit Hydroxyalkyl- oder (Poly)oxyalkylengruppen N,N-substituiertes p-Aminobenzoesäurederivat.

Aufgabe der vorliegenden Erfindung ist es, ein verbessertes Dentalmaterial für die direkte Zahnrestauration bereitzustellen, welches eine hohe DHT aufweist sowie eine hohe SBS und BF des gehärteten Restaurativs. Bevorzugt ist die gleichzeitige Einhaltung oder Verbesserung von Eigenschaften wie biologischer und toxikologischer Unbedenklichkeit, Lagerstabilität, Unempfindlichkeit gegenüber Umgebungslicht und Polymerisationsschrumpf sowie Farbstabilität, geringe Wasseraufnahme (WA) und Wasserlöslichkeit (WS) im ausgehärteten Zustand.

Die Erfindung löst diese Aufgabe durch die Merkmale der unabhängigen Patentansprüche. Vorteilhafte Ausführungsformen sind in den abhängigen Patentansprüchen angegeben.

Gegenstand der Erfindung ist ein Dentalmaterial, insbesondere ein lichthärtendes selbsthaftendes und selbstätzendes radikalisch polymerisierbares Dentalrestaurativ, das enthält:

a. wenigstens ein Monomer enthaltend mindestens eine radikalisch polymerisierbare ethylenisch ungesättigte Gruppe und mindestens eine Säuregruppe,

b. wenigstens ein Monomer, das keine Säuregruppen und mindestens eine radikalisch polymerisierbare ethylenisch ungesättigte Gruppe enthält,

c. wenigstens einen Photoinitiator,

d. mindestens ein tertiäres aromatisches Amin als Co-Initiator, das einen Benzolring aufweist, an den mindestens eine Dialkylamingruppe und mindestens eine weitere Gruppe direkt gebunden sind,

dadurch gekennzeichnet, dass die weitere Gruppe ausgewählt ist aus:

i. Carbonsäureestergruppen enthaltend mindestens eine Polyoxyalkylengruppe mit mindestens 2 Oxyethylen- und/oder Oxypropyleneinheiten, und

ii. Amidgruppen.

Die Erfindung stellt ein Dentalmaterial, bevorzugt Dentaladhäsiv, mit den in der Aufgabenstellung genannten Eigenschaften zur Verfügung, welches einfach und sicher mit wenigen Behandlungsschritten anzuwenden ist. Bevorzugt muss die Haftfläche (das Dentin) zuvor nicht mit Ätzmitteln und Haftvermittlern vorbereitet werden. Der Begriff polymerisierbares Dentalmaterial bezeichnet im Rahmen der Erfindung jegliche Dentalmaterialien, deren Aushärtung mindestens in wesentlichen Teilen durch (bevorzugt radikalische) Polymerisation erfolgt und die für eine oder bei einer dentalen Restauration verwendet werden können. Bevorzugt weist ein erfindungsgemäße Dentalmaterial wenigstens eine, weiter bevorzugt zwei, weiter bevorzugt alle drei der folgenden Eigenschaften auf: lichthärtend, selbsthaftend und selbstätzend. Es kann sich um ein Restaurationsmaterial oder um ein Adhäsiv handeln.

Ein die Anmelderin nicht bindender Erklärungsversuch der Wirkung der Erfindung ist, dass die verbesserte Haftung auf Dentin und die guten mechanischen Eigenschaften des erfindungsgemäßen Dentalmaterials (bei hoher Reaktivität) auf erhöhter Amphiphilie des Coinitiators beruhen, die einerseits eine sehr gute Löslichkeit in einer wässrigen Hybridschicht des Dentins als auch im Dentalmaterial selbst bewirkt, wobei das Dentalmaterial selbst vergleichsweise hydrophob ist.

Die Erfindung hat erkannt, dass hohe Anteile polymerisierbarer Säuren und HEMA gemäß dem Stand der Technik in einem dentalen Restaurationsmaterial verschiedene Nachteile haben, insbesondere können sie die Durchhärtetiefe (DHT) verringern und die Wasseraufnahme (WA) und Wasserlöslichkeit (WS) soweit erhöhen, sodass die Restaurationen nicht mehr biologisch und toxikologisch unbedenklich sind. Weiterhin ist es mit solchen Zusammensetzungen schwierig, die gewünschte Biegefestigkeit des Restaurationsmaterials zu erreichen.

Die bevorzugten radikalisch polymerisierbaren ethylenisch ungesättigten Gruppen sind die Acrylat-, die Methacrylat-, die Acrylamid- und die Methacrylamidgruppe. (Meth)acryl steht im Folgenden stets für beides - Acryl und Methacryl.

Geeignete polymerisierbare Säuren gemäß Merkmalsgruppe (a), d.h. Monomere enthaltend mindestens eine radikalisch polymerisierbare ethylenisch ungesättigte Gruppe und mindestens eine Säuregruppe sind dem Fachmann bekannt.

Bevorzugte Säuregruppen sind die Carbonsäure-, Phosphorsäure-, Phosphonsäure-, Bisphosphonsäure- und Sulfonsäuregruppe. Besonders bevorzugt sind die phosphorhaltigen Säuregruppen.

Exemplarisch seien genannt: 4-(Meth)acryloyloxyethyl-trimellitsäure; Bis-[2-(meth)acryloyloxyethyl]-phosphat; 10-(Meth)acryloyl-oxydecyl-dihydrogenphosphat (MDP)oder 2-Acrylamido-2-methylpropansulfonsäure.

Im Dentalmaterial ist die polymerisierbare Säure (a) bevorzugt zu 1 bis 20 Gew.-%, weiter bevorzugt zu 1 bis 15 Gew.-%, besonders bevorzugt zu 5 bis 15 Gew.-%, bezogen auf die Gesamtmasse der im Dentalmaterial enthaltenen Monomere enthaltend mindestens eine radikalisch polymerisierbare ethylenisch ungesättigte Gruppe, enthalten.

Im Dentalmaterial ist der Photoinitiator (c) bevorzugt zu 0,05 bis 10, bevorzugt zu 0,05 bis 5, am bevorzugtesten zu 0,1 bis 2 Gew.-% , bezogen auf die Gesamtmasse der im Dentalmaterial enthaltenen Monomere enthaltend mindestens eine radikalisch polymerisierbare ethylenisch ungesättigte Gruppe, enthalten.

Bevorzugte Photoinitiatoren (c) absorbieren verstärkt Licht im Wellenlängenbereich zwischen 390 und 560 nm, besonders bevorzugt zwischen 420 und 480 nm. Der Photoinitiator ist bevorzugt ein im Dentalmaterial lösliches aliphatisches 1,2-Diketon, wie Campherchinon (CQ) oder Propyl-phenyl-diketon (PPD), besonders bevorzugt jedoch Campherchinon und/oder ein Campherchinonderivat bspw. eine Carboxyl-, Carboxylat-, Sulfonsäure- oder Sulfonatgruppe aufweisend. Die Lichtabsorption im angegebenen Bereich löst einen initiierenden Übergang aus, der zur Radikalbildung im Photoinitiatorsystem führt.

Bevorzugt ist das tertiäre aromatische Amin (Coinitiator gemäß Merkmalsgruppe (d)) ausgewählt aus der Gruppe bestehend aus:

a)

Formel I

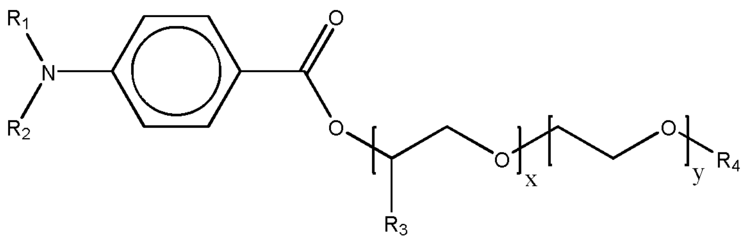

b)

Formel II

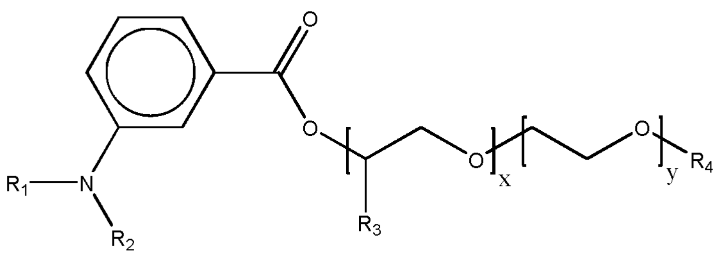

c)

Formel III

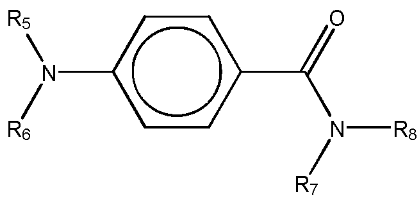

d)

Formel IV

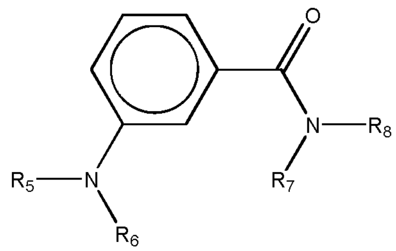

wobei in den Formeln bedeuten:

- $R_1$ und $R_2$ sind unabhängig voneinander) C1-C5-Alkyl, bevorzugt Methyl,
- $R_3$ ist Alkyl, bevorzugt Methyl,
- $R_4$ ist H oder C1-C5-Alkyl, bevorzugt Methyl,
- $R_5$ und $R_6$ sind unabhängig voneinander C1-C5-Alkyl, bevorzugt Methyl,
- $R_7$ und $R_8$ sind unabhängig voneinander ausgewählt aus H, Alkyl, Alkoxyalkyl und Polyalkylenoxid, bevorzugt Polyalkylenoxid mit mindestens zwei Oxyethylen- und/oder Oxypropyleneinheiten,

$x \geq 0$ und $y > 1$.

Die Summe aus x und y beträgt bevorzugt 3 bis 20, weiter vorzugsweise 5 bis 17. Bevorzugt ist $x < y$.

Für die Coinitiatoren (d) der Formeln I und II können die Polyalkylenoxidketten als Blockcopolymere oder als statistische Copolymere ausgebildet sein. Eine Polyalkylenoxidkette hat bevorzugt eine molare Masse zwischen 130 und 1200 g/mol, weiter bevorzugt zwischen 220 und 1200 g/mol, weiter bevorzugt zwischen 300 und 800 g/mol. Ein Beispiel ist 4-Dimethylamino-benzoesäure-poly(ethylenglykol-350)monomethylether-ester.

Geeignete Coinitiatoren (d) der Formeln III und IV sind 4-Dimethylaminobenzamid; 4-Dimethylamino-N-propylbenzamid; 3-Dimethylamino-N-propylbenzamid; 4-Dimethylamino-N-(hydroxyethyl)benzamid; 4-Dimethylamino-N-(hydroxypropyl)benzamid; 4-Dimethylamino-N-(hydroxybutyl)benzamid; 4-Dimethylamino-N-(2-methoxyethyl)benzamid; 3-Dimethylamino-N-(2-methoxyethyl)benzamid; 4-Dimethylamino-N-(2-(2-hydroxyethoxy)ethyl)benzamid. Besonders geeignete Coinitiatoren (d) der Formeln III und IV sind 4-Dimethylamino-N-(2-(2-(2-methoxyethoxy)ethoxy)ethyl)benzamid; 4-Dimethylamino-N-poly(ethylenoxid)methylether-benzamid; 4-Dimethylamino-N-poly(propylenoxid-block-ethylenoxid)methylether-benzamid; 4-Dimethylamino-N-poly(ethylenoxid-stat-propylenoxid)methylether-benzamid.

Die Polyalkylenoxidketten können als Blockcopolymere oder auch als statistische Copolymere verwendet werden. Eine Polyalkylenoxidkette hat besonders bevorzugt eine molare Masse zwischen 130 und 1200 g/mol.

Die erfindungsgemäßen Coinitiatoren ohne Amidgruppe weisen eine Wasserlöslichkeit größer > 2 g/l, weiter bevorzugt > 50 g/l, weiter bevorzugt > 100 g/l, weiter bevorzugt > 500 g/l auf.

Die erfindungsgemäßen Coinitiatoren mit Amidgruppe weisen eine Wasserlöslichkeit größer 0,4 g/l, bevorzugt > 2 g/l, weiter bevorzugt > 50 g/l, weiter bevorzugt > 100 g/l, weiter bevorzugt > 500 g/l auf.

Im Dentalmaterial ist der Coinitiator (d) zu 0,05 bis 10, bevorzugt zu 0,1 bis 10, am bevorzugtesten zu 0,5 bis 5 Gew.-%, bezogen auf die Gesamtmasse der im Dentalmaterial enthaltenen Monomere enthaltend mindestens eine radikalisch polymerisierbare ethylenisch ungesättigte Gruppe, enthalten.

Geeignete weitere Monomere (b), d.h. Monomere enthaltend mindestens eine radikalisch polymerisierbare ethylenisch ungesättigte Gruppe, die keine Säuregruppe aufweisen sind dem Fachmann bekannt.

Bevorzugt ist das wenigstens eine Monomer, das keine Säuregruppen und mindestens eine radikalisch polymerisierbare ethylenisch ungesättigte Gruppe enthält (Merkmal b) des Anspruchs 1) ausgewählt aus der Gruppe bestehend aus b1), b2) und b3), mit:

b1)

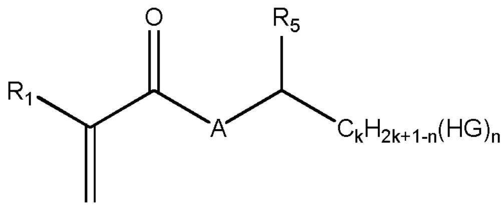

wobei in der Formel bedeutet:

$R^1$ ist H, Methyl oder Ethyl, bevorzugt H oder Methyl;

A ist O oder $NR^3$, mit $R^3$ = H, Methyl, Ethyl oder Propyl, bevorzugt $R^3$ = H;

HG ist eine hydrophile Gruppe ausgewählt aus OH und $(O\text{-}CH_2\text{-}CH_2)_m\text{-}OR^4$; bevorzugt ist HG = OH;

$R^5$ ist H, Methyl, Ethyl oder $C_kH_{2k+1-n}(HG)_n$; bevorzugt H;

$R^4$ ist H oder Methyl;

k, m, n sind natürliche Zahlen;

k = 1-5; bevorzugt 1-2;

m= 3-20; bevorzugt 5-20;

n= 1-5;

$n \leq k$;

jedes Kohlenstoffatom trägt höchstens eine hydrophile Gruppe HG;

b2) $(Q\ A\ E_v)_rC_tH_{(2t+2)-(r+s))}(HG)_s$
mit Q =

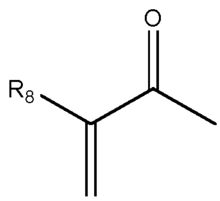

E =

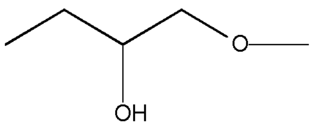

oder

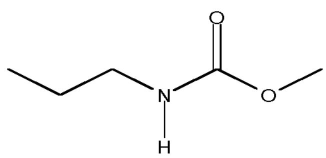

wobei in den Formeln bedeuten:

$R^8$ ist H, Methyl oder Ethyl, bevorzugt H oder Methyl; A ist O oder $NR^3$, mit $R^3$ = H, Methyl, Ethyl oder Propyl, bevorzugt $R^3$ = H;
HG ist OH, $(O\text{-}CH_2\text{-}CH_2)_m\text{-}OR^7$; bevorzugt OH;
$R^7$ ist H oder Methyl;

m, r, s, t, v sind natürliche Zahlen;
m= 3-20, bevorzugt 6-20;
r= 2-4;
s= 1-4;
t= 2-8; bevorzugt 2-6;
v= 0 oder 1; bevorzugt 0.

b3) $Q\text{-}A\text{-}E_v\text{-}C_2H_2\text{-}HG\text{-}E_v\text{-}A\text{-}Q$
mit Q =

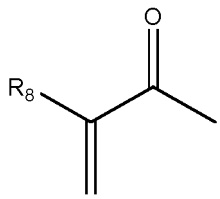

E =

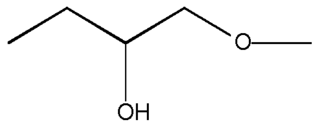

oder

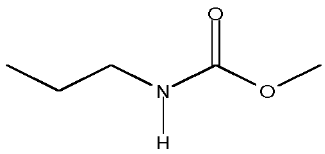

wobei in den Formeln bedeuten:

$R^8$ ist H, Methyl oder Ethyl, bevorzugt H oder Methyl;
A ist O oder $NR^3$, mit $R^3$ = H, Methyl, Ethyl oder Propyl, bevorzugt $R^3$ = H;
HG ist $(O\text{-}CH_2\text{-}CH_2)_m$;
m= 4-20, bevorzugt 6-20;
v= 0 oder 1; bevorzugt 0.

Bevorzugte Monomere b1) wie oben beschrieben sind dem Fachmann bekannt. Beispiele sind Hydroxyethylmethacrylat, Hydroxypropylmethacrylat, Hydroxyethylacrylamid, Hydroxypropylacrylamid, 2,3-Dihydroxypropylmethacrylat, 1,3-Dihydroxy-2-propanylmethacrylate, Methoxy(polyethylenglycol-350)methacrylat oder Methoxy(polyethylenglycol-500)methacrylat. Besonders bevorzugt ist Hydroxyethylmethacrylat (HEMA).

Bevorzugte Monomere b2) wie oben beschrieben sind dem Fachmann bekannt. Beispiele sind Glyceroldimethacrylat und N,N'-(1,2-Dihydroxyethylen)bisacrylamid. Weitere bevorzugte Monomere sind in der EP2133368 (Sekiguchi) beschrieben.

Bevorzugte Monomere b3) wie oben beschrieben sind dem Fachmann bekannt. Beispiele sind Polyethylenglykol 200 Di(meth)acrylat; Polyethylenglykol 300 Di(meth)acrylat; Polyethylenglykol 400 Di(meth)acrylat; Polyethylenglykol 600 Di(meth)acrylat; PPGDMA, Polypropylenglykoldimethacrylat.

Geeignete Monomere b1) bis b3) weisen bevorzugt mindestens eine Wasserlöslichkeit von 50 g/l Wasser auf.

Das erfindungsgemäße Dentalmaterial kann bevorzugt zusätzlich wenigstens ein Monomer b4), das keine Säuregruppen und mindestens zwei radikalisch polymerisierbare ethylenisch ungesättigte Gruppen enthält, aufweisen, wobei dieses wenigstens eine Monomer b4) nicht aus der Gruppe b1), b2) und b3) ausgewählt ist.

Bevorzugte Monomere b4) wie oben beschrieben sind dem Fachmann bekannt. Beispiele sind Allyl(meth)acrylat; 1,3-Propandioldi(meth)acrylat; 1,3-Butandioldi(meth)acrylat; 1,4-Butandioldi(meth)acrylat; 1,5-Pentandioldi(meth)acrylat; Neopentylglykoldi(meth)acrylat; 1,6-Hexandioldimethacrylat; 1,9-Nonandioldi(meth)acrylat; 1,10-Decandioldi(meth)acrylat; 1,12-Dodecandioldi(meth)acrylat; Ethylenglykoldi(meth)acrylat; Diethylenglykoldi(meth)acrylat; Triethylenglykoldi(meth)acrylat; Tetraethylenglykoldi(meth)acrylat; Propoxyliertes (2) Neopentylglykoldi(meth)acrylat; Bisphenol-A-di(meth)acrylat; Bisphenol-A-glyceroldi(meth)acrylat (BisGMA); Ethoxyliertes Bisphenol-A-Di(meth)acrylat; Propoxyliertes Bisphenol-A-Di(meth)acrylat; Diurethandi(meth)acrylat (UDMA); Tricyclo[5.2.1.0]decan-dimethanoldi(meth)acrylate; N,N'-Ethylenbisacrylamid; N,N'-Diethyl(1,3-propylen)bisacrylamid; N,N'-(2,2,4-Trimethyl-hexamethylen)-bismethacrylamid; Bis[2-(2-methylacryl-amino)-ethoxycarbonyl]-hexamethylendiamin; Trimethylolpropan-tri(meth)acrylat; Di-Trimethylolpropan-tetra(meth)acrylat; Di-Pentaerythritolpenta(meth)-acrylat; Di-Pentaerythritolhexa(meth)acrylat.

Die Wasserlöslichkeit des Monomers b4) beträgt bevorzugt weniger als 50 g/l.

Besonders bevorzugt sind Mischungen von mindestens einem Monomeren b1) bis b3) und mindestens einem Monomeren bzw. Vernetzer der Gruppe b4).

Das mittels Bestrahlung mit sichtbarem Licht radikalisch polymerisierbare Dentalmaterial enthält weiterhin bevorzugt 0,1 bis 90 Gew.-%, bevorzugt 45 bis 70 Gew.-% organische und/oder anorganische Füllstoffe.

Geeignete Füllstoffe sind dem Fachmann bekannt. Bevorzugt sind oberflächenbehandelte anorganische Füllstoffe, die gegenüber den Säuren einer Harzmatrix im Wesentlichen inert sind, z.B. die im Dentalmaterial, d.h. der Harzmatrix unlöslich sind, wobei unter Harzmatrix die radikalisch polymerisierbaren Monomere und darin gelöste Zusätze verstanden werden. Geeignete Füllstoffe werden beispielsweise in EP 1 720 506 (Neffgen et al) offenbart.

Das mittels Bestrahlung mit sichtbarem Licht radikalisch polymerisierbare Dentalmaterial enthält weiterhin bevorzugt 0,01 bis 20, bevorzugt 0,01 bis 10 Gew.-% dentalübliche Additive.

Die Auswahl der Additive nimmt der Fachmann in Abhängigkeit von der vorgesehenen Verwendung des Dentalmaterials vor. Übliche dentale Additive sind Polymerisationsinhibitoren, wie BHT, UV-Stabilisatoren, Rheologiemodifizierer, Farbstoffe und Röntgenopaker.

Das mittels Bestrahlung mit sichtbarem Licht radikalisch polymerisierbare Dentalmaterial kann weiterhin nicht polymerisierbare organische Lösungsmittel, beispielweise leicht flüchtige Lösungsmittel wie Ethanol oder Aceton, sowie Wasser enthalten.

Gegenstand der Erfindung ist ferner eine erfindungsgemäße Zusammensetzung zur Verwendung als ein lichthärtendes selbsthaftendes und selbstätzendes radikalisch polymerisierbares Dentalrestaurativ bzw. die Verwendung einer solchen Zusammensetzung als oder bei der Herstellung eines lichthärtenden selbsthaftenden und selbstätzenden radikalisch polymerisierbaren Dentalrestaurativs.

Ein weiterer Gegenstand der Erfindung ist die Verwendung eines tertiären aromatisches Amins, das einen Benzolring aufweist, an den mindestens eine Dialkylamingruppe und mindestens eine weitere Gruppe direkt gebunden sind, wobei die weitere Gruppe ausgewählt ist aus:

i. Carbonsäureestergruppen enthaltend mindestens eine Polyoxyalkylengruppe mit mindestens 2 Oxyethylen- und/oder Oxypropyleneinheiten, und

ii. Amidgruppen

als Coinitiator in einem lichthärtenden selbsthaftenden und selbstätzenden radikalisch polymerisierbaren Dentalrestaurativ.

Ausführungsbeispiele der Erfindung werden nachfolgend erläutert.

Verwendete Materialien:

| | |
|---|---|
| 10-Methacryloyl-oxydecyl-dihydrogenphosphat (MDP) | PCM Products |
| 2-(2-Aminoethoxy)ethanol | Alfa Aeser |
| 2,6-Di-tert-butyl-4-methylphenol (BHT) | Merck |
| 2-Hydroxyethylmethacrylat (HEMA) | Melrob (Evonik) |
| 2-Methoxyethylamin | Merck |
| 3-Dimethylaminobenzoesäure | Merck |
| 3-Trimethoxysilylpropylmethacrylat (MEMO) | Evonik |
| 4-Dimethylaminobenzoesäure | Alfa Aeser |
| 4-Dimethylaminobenzoesäure-2-ethylhexylester (EHA) | Rahn |
| 4-Dimethylaminobenzoesäure-ethylester | Sigma Aldrich |
| 4-Dimethylaminobenzoesäure-2-n-butoxyethylester | |

(fortgesetzt)

| | |
|---|---|
| 4-Dimethylaminobenzonitril | Alfa Aeser |
| 4-Dimethylaminobenzoylchlorid | Alfa Aeser |
| Aktivkohle | Merck |
| $Al_2O_3$ DC-Karten (Polygram Alox N/UV $_{254}$) | Macherey-Nagel |
| $Al_2O_3$ für Säulenchromatographie (Aluminiumoxid 90 aktiv, neutral) | Merck |
| $BaCO_3$ | Sigma Aldrich |
| Bisphenol-A-diglycidyldimethacrylat (BisGMA) | CCP Composites |
| $CaCl_2$, wasserfrei | Sigma Aldrich |
| Campher-10-sulfonsäure | Merck |
| Celite 545 | Merck |
| $CuSO_4$ x 5 $H_2O$ | Merck |
| D,L-Campherquinon (CQ) | Rahn |
| Dibenzo-18-krone-6 | Sigma Aldrich |
| Dichlormethan | Merck |
| Diethylether | Carl Roth |
| Dimethylsulfoxid | Merck |
| Dowex 50 WX 8 | Fluka |
| Essigsäure | Merck |
| Ethanol | Walter CMP |
| Ethylacetat | Merck |
| Ethyldiisopropylamin | Acros |
| Glaspulver BAF GM 27884 0,7 $\mu$m, mit 3-Trimethoxysilylpropylmethacrylat silanisiert | Schott |
| $H_2O_2$, 35 % | Sigma Aldrich |
| HCl | Merck |
| Hydrophobe, hochdisperse Kieselsäure (HDKH 2000) | Wacker |
| Jeffamin M 1000 | Huntsman |
| $K_2CO_3$ | J.T. Baker |
| $K_3PO_4$ | Alfa Aeser |
| Methanol | Sigma Aldrich |
| $Na_2S_2O_5$ | Sigma Aldrich |
| $Na_2SO_3$ | Merck |
| $Na_2SO_4$ | Merck |
| $NaBH_4$ | Merck |
| NaCl | Merck |
| $NaHCO_3$ | J.T. Baker |
| $NaN_3$ | Merck |
| NaOH | Merck |
| Natriumethoxid-Lösung, 21% in Ethanol | Sigma Aldrich |
| n-Heptan | Merck |

(fortgesetzt)

| | |
|---|---|
| n-Propylamin | Sigma Aldrich |
| O-(Benzotriazol-1-yl)-N,N,N',N'-tetramethyluronium tetrafluoroborat (TBTU) | Iris Biotech |
| Polyethylenglykolmonomethylether 350 | Sigma Aldrich |
| p-Toluolsulfonsäurechlorid | Fluka |
| Schwefelsäure | Sigma Aldrich |
| Selendioxid | Merck |
| $SiO_2$ DC-Karten (Polygram Sil G/UV $_{254}$) | Macherey-Nagel |
| $SiO_2$ für Mitteldruckchromatographie (Kieselgel 60, 0,04-0,063 mm) | Merck |
| $SiO_2$ für Säulenchromatographie (Kieselgel 60, 0,063-0,200 mm) | Merck |
| Tetrahydrofuran (THF) | Sigma Aldrich |
| Tetrahydrofuran (THF), wasserfrei | Sigma Aldrich |
| Toluol | Merck |
| Triethylenglykoldimethacrylat (TEDMA) | Lehmann & Voss |
| Triethylenglykolmonomethylether | Merck |
| VE-Wasser | DMG |

**Synthesen**

Campherchinon-10-sulfonsäure

In einem Einhalskolben mit Magnetrührfisch werden 3,15 g (28,4 mmol) Selendioxid in 20 ml VE-Wasser gelöst. Dazu werden 10,6 g (45,6 mmol) Campher-10-sulfonsäure gegeben und der Kolben mit einem Rückflusskühler ausgestattet. Das Reaktionsgemisch wird auf 110 °C unter Rückflusskühlung erhitzt. Im Abstand von 3 h werden drei weitere Portionen von jeweils 3,15 g Selendioxid zum Ansatz gegeben. Nach der letzten Zugabe wird für 4,5 h bei 110 °C unter Rückflusskühlung erhitzt. Nach Abkühlung auf Raumtemperatur wird der Ansatz über Celite 545 filtriert und der Filterrückstand mit 2x50 ml VE-Wasser gewaschen. Die Lösung wird mit weiteren 50 ml VE-Wasser verdünnt, sowie mit 2 ml einer 20 %-i-gen Schwefelsäure-Lösung versetzt und in einen Zweihalskolben überführt.

In einer Gasentwicklungsapparatur wird aus 63 g $Na_2SO_3$ und 100 ml einer 6 M $H_2SO_4$ innerhalb von einer Stunde Schwefeldioxid entwickelt und durch eine Kapillare in den filtrierten Ansatz geleitet. Der rotbraune Ansatz rührt nach beendeter Gaseinleitung über Nacht. Danach wird die oben beschriebene Gasentwicklung und Einleitung wiederholt. Der rotbraun-schwarze Ansatz wird über Celite 545 filtriert und der Filterrückstand mit VE-Wasser gewaschen. Vom Ansatz werden bei 100 mbar ca. 130 ml Wasser abdestilliert. Der Destillationsrückstand wird mit festem $BaCO_3$ neutralisiert. Die dickflüssige rötlich-braune Masse wird über Celite 545 filtriert und der Filterrückstand mit ca. 150 ml VE-Wasser gewaschen. Eine klare, gelbe Lösung wird erhalten. Die Lösung wird am Rotationsverdampfer eingeengt, bis ca. 80 ml Flüssigkeit übrig bleiben. Dabei entsteht ein weißer Niederschlag, der abfiltriert wird. Die klare, gelbe Lösung wird über eine Säule (h = 12 cm, d = 2 cm) mit Ionenaustauscher (Dowex 50WX8, H-Form) gegeben. Nachdem die Lösung vollständig in die Säule eingezogen ist, wird die Säule mit VE-Wasser gespült, bis die austretende Flüssigkeit aus der Säule farblos ist. Die gelbe Lösung wird am Rotationsverdampfer bis zur Trockene eingedampft und der Rückstand im Vakuum bei Raumtemperatur getrocknet. Das Rohprodukt (ca. 10,9 g) wird in 100 ml Methanol gelöst. Zu der Lösung werden ca. 2 g Aktivkohle gegeben und die Suspension wird zwei Stunden unter Rückflusskühlung erhitzt. Nach Abkühlung auf Raumtemperatur wird über Celite 545 filtriert und der Filterrückstand mit 3x20 ml Methanol gewaschen. Man erhält eine klare, hellgelbe Lösung. Methanol wird am Rotationsverdampfer entfernt. Nach Trocknung im Vakuum verbleibt eine hochviskose, hellgelbe Flüssigkeit, die über Nacht im Kühlschrank auskristallisiert. Der gelbe Feststoff wird aus Ethylacetat/n-Heptan umkristallisiert. Ausbeute: 6,02 g. $^1$H-NMR ($d_6$-DMSO) ppm: 0,82 (s, 3H), 0,11 (s, 3H), 1,50 (m, 1H), 1,65 (m, 1H), 2,13 (m, 1H), 2,59 (d, 1H), 2,65 (d, 1H), 2,75 (dt, 1H), 2,95 (d, 1H), Schmelzpunkt: 155 °C. Als Verunreinigung sind ca. 10 % des Edukts Campher-10-sulfonsäure vorhanden

Natrium-Campherchinon-10-sulfonat

1,01 g (4,1 mmol) der Campherchinon-10-sulfonsäure werden in 0,5 ml VE-Wasser gelöst. Die Lösung wird in einem Eisbad gekühlt. Mit einer Eppendorf-Pipette werden 2 ml einer 2N NaOH-Lösung langsam zugetropft. Das Eisbad wird entfernt und die Lösung wird drei Stunden bei Raumtemperatur gerührt. Am Rotationsverdampfer wird das Wasser abdestilliert. Der Rückstand wird im Vakuum über $CaCl_2$ getrocknet. Das Produkt fällt als gelber Feststoff an. Ausbeute: 0,99 g, Schmelzpunkt: 250 °C (Zersetzung).

4-Dimethylaminobenzoesäure-poly(ethylenglykol)-monomethyl-ether-esters

In einem Zweihalskolben mit Magnetrührfisch werden 35 g Polyethylenglykolmonomethylether (100 mmol) und 29 g (150 mmol) 4-Dimethylaminobenzoesäureethylester gegeben. Dazu werden 2 ml einer Natriumethoxid-Lösung (21 Gew. % in Ethanol (5 mmol)) zugefügt. Der Kolben wird mit einem Hahn und Stopfen verschlossen und der Inhalt unter Vakuum (750 mbar) gesetzt. Der Kolben wird in ein vortemperiertes Ölbad (100 °C) gehängt und das Vakuum wird schrittweise auf 40 mbar runtergeregelt. Nach zwei Stunden wird das Vakuum gebrochen und der Ansatz auf ca. 50 °C abgekühlt. Dann werden erneut 2 ml der Natriumethoxid-Lösung (5 mmol in Ethanol) zugefügt. Es wird wieder ein leichtes Vakuum von 750 mbar angelegt und der Kolben in ein 100 °C warmes Ölbad überführt. Das Vakuum wird dann schrittweise auf 40 mbar reduziert und der Ansatz drei Stunden bei diesen Bedingungen im Ölbad belassen. Danach wird der Ansatz auf Raumtemperatur gebracht und das Vakuum gebrochen. Zur Aufarbeitung wird der Ansatz in eine Mischung aus 500 ml VE-Wasser und 5 ml einer 2N HCl-Lösung gegeben. Es entsteht ein weißer Niederschlag. Der Feststoff wird abfiltriert. Zum Filtrat wird 1 ml einer gesättigten Natriumbisulfit-Lösung gegeben und mit einer 2N NaOH-Lösung der pH-Wert auf ca. 7,5 eingestellt. Die gelblich-grüne Emulsion wird mit 6,3 g Aktivkohle versehen und eine Stunde auf einer Rüttelplatte geschüttelt. Die Suspension wird über Celite 545 filtriert. Die wässrige Lösung wird mit 3x150 ml Dichlormethan extrahiert. Die organische Phase wird über $Na_2SO_4$ getrocknet. Das Trocknungsmittel wird abgetrennt und das Lösungsmittel am Rotationsverdampfer entfernt. Nach Trocknung im Vakuum fällt das Produkt als schwach gelbliches Öl an. Ausbeute 28,2 g, $^1$H-NMR ($CDCL_3$) ppm: 3,00 (s, 6H), 3,35 (s, 3H), 3,52 (t, 2H), 3,56-3,75 (br, ca. 30H), 3,79 (t, 2H), 4,39 (t, 2H), 6,61 (d, 2H), 7,89 (d, 2H)

4-Dimethylaminobenzoesäure-tri(ethylenglykol)monomethyl-ether-esters

In einem Einhalskolben mit Magnetrührfisch, Tropftrichter und $CaCl_2$-Trockenrohr werden bei Raumtemperatur 0,92 g (5 mmol) 4-Dimethylaminobenzoylchlorid, 20 ml wasserfreies THF und 1,16 ml (7 mmol) Diisopropylethylamin vorgelegt. Die Lösung wird im Eisbad auf 0 °C gekühlt. Im Tropftrichter wird eine Lösung aus 5 ml wasserfreiem THF und 0,78 ml (4,9 mmol) Triethylenglykolmonomethylether zubereitet. Diese Lösung wird langsam unter Eiskühlung zum Reaktionsansatz getropft. Nach Zugabe lässt man den Ansatz auf Raumtemperatur kommen. Der Ansatz wird ca. 70 h unter Feuchtigkeits- und Lichtausschluss bei Raumtemperatur gerührt. Hierbei entsteht etwas Niederschlag. Zur Aufarbeitung wird die Lösung vom Niederschlag dekantiert. Der Niederschlag wird mit etwas THF gewaschen. Die organischen Phasen werden vereinigt und das Lösungsmittel am Rotationsverdampfer entfernt. Der Rückstand wird in 50 ml Dichlormethan aufgenommen. Die organische Phase wird mit 2x10 ml 0,5 N HCl-Lösung, 1x10 ml gesättigter $NaHCO_3$-Lösung und 1x10 ml gesättigter NaCl-Lösung ausgeschüttelt. Anschließend wird die organische Phase über $Na_2SO_4$ getrocknet. Nach Filtration wird das Lösungsmittel am Rotationsverdampfer entfernt und der Rückstand im Hochvakuum getrocknet. Es fallen 1,47 g Rohprodukt an. Das Rohprodukt wird durch Säulenchromatographie weiter aufgereinigt. Stationäre Phase: $SiO_2$. Laufmittel: Ethylacetat/n-Heptan 1:1. Säule: h = 35 cm, d = 4 cm. Das Produkt hat einen $R_f$ = 0,17. Das Produkt fällt als klares, blassgelbliches Öl an. Ausbeute: 0,75 g. $^1$H-NMR ($CDCL_3$) ppm: 3,00 (s, 6H), 3,34 (s, 3H), 3,50 (m, 2H), 3,60-3,70 (br, ca. 6H), 3,78 (t, 2H), 4,39 (t, 2H), 6,60 (d, 2H), 7,89 (d, 2H)

4-Dimethylaminobenzamid

In einem Zweihalskolben mit Magnetrührfisch, Tropftrichter und Rückflusskühler werden 2,31 g (15,8 mmol) umkristallisiertes 4-Dimethylbenzonitril in 10 ml DMSO gelöst. Zu der hellbraunen Lösung werden 0,32 g (2,3 mmol) $K_2CO_3$ gegeben. Innerhalb von 5 min werden 1,9 ml einer 35 %-igen $H_2O_2$-Lösung (18,6 mmol in Wasser) bei Raumtemperatur langsam zugetropft. Dabei fällt ein weißer Feststoff aus. Nach Zugabe werden weitere 45 min bei Raumtemperatur unter Rückflusskühlung gerührt. Während dieser Phase steigt kurzzeitig die Temperatur an. Es bildet sich so viel weißer Feststoff, dass der Magnetrührfisch sich nicht mehr bewegt. Zur Aufarbeitung werden 50 ml VE-Wasser zum Ansatz gegeben und mit dem Spatel wird der Brei homogenisiert. Der Feststoff wird über eine Glasfritte abgetrennt und mit 3x20 ml VE-Wasser gewaschen. Im Exsikkator wird der Feststoff im Vakuum bei Raumtemperatur über $CaCl_2$ getrocknet. Das Produkt fällt als weißer Feststoff an. Ausbeute: 2,42 g. $^1$H-NMR ($d_6$-DMSO) ppm: 2,95 (s, 6H), 6,67 (d, 2H), 6,89 (br. s, NH), 7,60 (br. s, NH), 7,73 (d, 2H)

4-Dimethylamino-N-poly(propylenoxid-block-ethylenoxid)-methylether-benzamids

In einem Dreihalskolben mit Magnetrührfisch, Tropftrichter, Rückflusskühler und Hahn werden 4,25 g (20 mmol) gemörsertes, wasserfreies $K_3PO_4$ vorgelegt. Der Kolben wird mit Stickstoff gespült und die gesamte Apparatur mit einer Heißluftpistole ausgeheizt. Im $N_2$-Strom wird auf Raumtemperatur abgekühlt. Dann werden 80 ml wasserfreies THF zum $K_3PO_4$ gegeben. Zur Suspension werden 2,94 g (16 mmol) 4-Dimethylamino-benzoylchlorid und 0,04 g (0,1 mmol) Dibenzo-18-krone-6 zugefügt. Eine Lösung aus 8,04 g Jeffamin M 1000 in 12 ml wasserfreiem THF wird zubereitet und in den Tropftrichter überführt. Unter Ausschluss von Feuchtigkeit wird die Jeffamin M 1000-Lösung bei Raumtemperatur innerhalb von 15 min zum Ansatz gegeben. Dann wird ein Ballon, befüllt mit $N_2$, auf die Apparatur gesteckt. Tagsüber wird der Ansatz unter Schutzgas und Rückflusskühlung für 5 bis 8 h im Ölbad auf 50 °C erwärmt. Die sonstige Zeit wird bei Raumtemperatur gerührt. Insgesamt wird der Ansatz für 44 h bei 50°C und für 148 h bei Raumtemperatur gerührt. Zur Aufarbeitung wird der Ansatz filtriert. Der Filterrückstand wird mit etwas THF gewaschen. Das Lösungsmittel wird am Rotationsverdampfer entfernt und der Rückstand im Hochvakuum von flüchtigen Bestandteilen befreit. Das Rohprodukt wird in eine Mischung aus 80 ml VE-Wasser und 0,8 ml 2 N HCl gegeben. Die sich bildende Emulsion wird nochmals mit 100 ml VE-Wasser verdünnt und filtriert. Zur filtrierten Lösung werden 0,5 ml gesättigte Natriumbisulfit-Lösung zugegeben und mit einer 2 N NaOH auf einen pH-Wert von 7,5 eingestellt. In einem Scheidetrichter wird die wässrige Phase mit 3x60 ml Dichlormethan extrahiert. Die vereinigten organischen Phasen werden über $Na_2SO_4$ getrocknet. Zur extrahierten wässrigen Phase wird festes NaCl gegeben, woraufhin sich eine organische Phase absetzt. Diese wird abgetrennt und ebenfalls über $Na_2SO_4$ getrocknet. Alle getrockneten organischen Phasen werden vereinigt und das Lösungsmittel im Vakuum am Rotationsverdampfer entfernt. Das Produkt fällt als leicht beiger, wachsartiger Feststoff an. Ausbeute: 5,94 g. $^1$H-NMR ($CDCl_3$) ppm: 1,04 (m, ca. 5H), 1,17 (m, ca. 3H), 1,76 (m, ca. 1H), 2,93 (s, 6H), 3,29 (s, ca. 3H), 3,31 - 3,51 (br, ca. 10H), 3,56 (s, ca. 78H), 6,34 (d, NH), 6,58 (d, ca. 2H), 7,67 (m, ca. 2H)

4-Dimethylamino-N-(2-(2-hydroxyethoxy)ethyl)-benzamids

In einem Einhalskolben mit Magnetrührfisch und Stopfen wurden 6,61 g (40 mmol) 4-Dimethylaminobenzoesäure in 200 ml Dichlormethan suspendiert und durch Zugabe von 13,22 ml (80 mmol) Ethyldiisopropylamin gelöst. Zu der klaren Lösung wurden 12,85 g (40 mmol) TBTU in Portionen bei Raumtemperatur gegeben. Nach beendeter Zugabe wurde der Kolben mit einem Stopfen verschlossen und 14 h bei Raumtemperatur gerührt. Es entstand eine gelbe, leicht trübe Lösung. In einem 500 ml Vierhalskolben mit zwei Stopfen, Innenthermometer, Magnetrührfisch und Tropftrichter mit $CaCl_2$-Trockenrohr wurden 4 ml (40 mmol) 2-(2-Aminoethoxy)-ethanol in 100 ml Dichlormethan gelöst. Durch ein Eisbad wurde die Lösung auf 0 °C gekühlt. Das Gemisch aus 4-Dimethylaminobenzoesäure, Ethyldiisopropylamin und TBTU in Dichlormethan wurde in den Tropftrichter überführt und langsam, innerhalb von 90 min zur 2-(2-Aminoethoxy)ethanol-Lösung zugegeben, wobei die Innentemperatur nicht über 5 °C anstieg. Nach beendeter Zugabe wurde das Eisbad entfernt und das Reaktionsgemisch 48 h bei Raumtemperatur gerührt. Zur Aufarbeitung wurde das Reaktionsgemisch in einen Scheidetrichter überführt und mit 4x25 ml 0,5 N Essigsäure-, 2x25 ml gesättigter $NaHCO_3$- und 1x25 ml gesättigter NaCl-Lösung extrahiert. Die organische Phase wurde über $Na_2SO_4$ getrocknet. Das Lösungsmittel wurde am Rotationsverdampfer entfernt. Zum Rückstand wurden 180 ml Diethylether gegeben. Dabei löste sich nicht alles. Vom Feststoff wurde die klare Diethylether-Phase abdekantiert. Das Lösungsmittel wurde am Rotationsverdampfer entfernt. Ausbeute Rohprodukt: 12,12 g. Zur weiteren Aufreinigung wurde das Rohprodukt in 70 ml einer Mischung aus Ethylacetat/Ethanol 10:2 gelöst. Dazu wurden 65 g $Al_2O_3$ gegeben und das Lösungsmittelgemisch am Rotationsverdampfer entfernt. Das auf Al2O3 aufgezogene Rohprodukt wurde auf eine mit 100 g $Al_2O_3$ befüllte Säule (d = 3 cm) gegeben und das Rohprodukt durch Säulenfiltration aufgereinigt. Laufmittel: Ethylacetat/Ethanol 10:2. Das Laufmittel wurde am Rotationsverdampfer entfernt und der Rückstand im Vakuum von flüchtigen Bestandteilen befreit. Ausbeute aufgereinigtes Rohprodukt: 9,24 g.

2-(2-(2-Methoxyethoxy)ethoxy)ethyl-tosylat

18,73 g (114 mmol) Methoxytriethylenglykol wurden in einem Dreihalskolben vorgelegt und in 60 ml THF gelöst. Mittels eines Eisbades wurde die Lösung auf ca. 0 °C gekühlt. Zu der Lösung wurden 35 ml einer 6 N NaOH-Lösung zugegeben.

Dabei erwärmte sich die Mischung auf 8 °C. Nachdem die Innentemperatur wieder bei ca. 0 °C lag, wurde eine Lösung von 40,4 g (212 mmol) p-Toluolsulfonyl-chlorid in 70 ml THF zur Lösung unter starkem Rühren getropft, wobei die Innentemperatur unter 5 °C gehalten wurde. Nach beendeter Zugabe wurde 30 min bei ca. 0 °C gerührt. Danach wurde das Eisbad entfernt und der Ansatz wurde 45 min bei Raumtemperatur gerührt. Zur Aufarbeitung wurde der Ansatz auf 300 ml Diethylether gegeben. Dazu wurden 50 ml einer 1 NaOH-Lösung gegeben und extrahiert. Die organische Phase wurde abgetrennt und nochmals mit 50 ml einer 1 N NaOH-Lösung extrahiert. Nach erneuter Abtrennung der organischen Phase wurde diese über $Na_2SO_4$ getrocknet und das Lösungsmittel am Rotationsverdampfer entfernt.

Ausbeute Rohprodukt: 44,91 g.

Das Rohprodukt wurde durch Säulenchromatographie weiter aufgereinigt. Dazu wurden ca. 5 g auf eine Kieselgelsäule aufgebracht (Säule: d = 3 cm, stationäre Phase 75 g $SiO_2$). Zunächst wurde mit dem Laufmittelgemisch n-Heptan/Ethylacetat 10:1 eluiert (ca. 1,2 l), bis durch Dünnschichtchromatographie kein Edukt (p-Toluol-sulfonylchlorid) mehr nachgewiesen konnte. Dann wurde das Laufmittelgemisch umgestellt auf n-Heptan/Ethylacetat 1:1 und das Produkt eluiert. Das Lösungsmittel wurde am Rotationsverdampfer entfernt und der Rückstand im Vakuum von flüchtigen Bestandteilen befreit. Ausbeute: 4,21 g. $^1$H-NMR ($CDCl_3$) ppm: 2,42 (s, 3H), 3,35 (s, 3H), 3,51 (m, 2H), 3,57-3,60 (m, 6H), 3,64-3,67 (t, 2H), 4,11-4,15 (t, 2H), 7,32 (d, 2H), 7,77 (d, 2H)

2-(2-(2-Methoxyethoxy)ethoxy)ethyl-azid

In einem verschraubbaren Reagenzglas mit Magnetrührfisch wurden 0,45 g (6,9 mmol) $NaN_3$ in 1 ml VE-Wasser suspendiert. Dazu wurde eine Lösung von 1,50 g (4,7 mmol) 2-(2-(2-Methoxyethoxy)-ethoxy)ethyl-tosylat in 0,5 ml THF gegeben. Der Ansatz wurde verschlossen und 7,5 h unter starkem Rühren im Ölbad auf 70 °C erhitzt. Der Ansatz wurde zur Aufarbeitung auf 15 ml VE-Wasser gegeben und mit 4x25 ml Diethylether extrahiert. Die organische Phase wurde über $Na_2SO_4$ getrocknet und anschließend wurde das Lösungsmittel am Rotationsverdampfer entfernt. Der Rückstand wurde im Vakuum von flüchtigen Bestandteilen befreit. Ausbeute: 0,71 g. $^1$H-NMR ($CDCl_3$) ppm: 3,31-3,34 (m, 5H), 3,47-3,50 (m, 2H), 3,58-3,63 (m, 8H)

2-(2-(2-Methoxyethoxy)ethoxy)ethyl-amin

In einem Kolben wurden 1,54 g (6,17 mmol) $CuSO_4$ x 5 $H_2O$ in 180 ml Methanol gelöst. Die Lösung wurde in einem Eisbad auf 0 °C gekühlt. Unter Rühren wurden 0,69 g $NaBH_4$ zur Lösung gegeben. Eine starke Gasentwicklung und eine leichte Erwärmung setzten ein und es entstand eine schwarze Suspension. 11,68 g (61,7 mol) 2-(2-(2-Methoxyethoxy)ethoxy)-ethyl-azid gelöst in 90 ml Methanol wurden zur schwarzen Suspension gegeben. Bei 0 °C wurden innerhalb von 3,75 h 6x412 mg (6 x 10,9 mmol) $NaBH_4$ zum Ansatz gegeben. Nach der letzten Zugabe wurde 30 min bei 0 °C nachgerührt. Zur Aufarbeitung wurde der Ansatz mit 2 N NaOH-Lösung auf pH = 12 eingestellt. Dabei entstand ein Niederschlag, der über eine Fritte mit einer dünnen Schicht Celite 545 abfiltriert wurde. Der Rückstand wurde mit etwas Methanol gewaschen. Die Methanol-Lösung wurde über Nacht stehen gelassen, es bildete sich wieder etwas Niederschlag. Methanol wurde am Rotationsverdampfer entfernt und der Rückstand in 800 ml Dichlormethan aufgenommen. Die organische Phase wurde über $Na_2SO_4$ getrocknet. Das Lösungsmittel wurde am Rotationsverdampfer entfernt und der Rückstand im Vakuum von flüchtigen Bestandteilen entfernt. Der Rückstand wurde in 30 ml Toluol aufgenommen, filtriert und mit weiteren 200 ml Toluol versetzt. Ca. 70 ml Toluol wurden abdestilliert, um das Rohprodukt azeotrop zu trocknen. Restliches Toluol wurde am Rotationsverdampfer entfernt und der Rückstand im Vakuum von flüchtigen Bestandteilen befreit. Das Rohprodukt (8,12 g) wurde durch eine Destillation im Kugelrohr weiter aufgereinigt. Das Produkt ging bei 5 mbar zwischen 160 und 200 °C als farbloses Öl über. Ausbeute: 6,58 g. $^1$H-NMR ($CDCl_3$) ppm: 2,82 (t, 2H), 3,33 (s, 3H), 3,47-3,51 (m, 4H), 3,58-3,61 (m, 6H)

4-Dimethylamino-N-(2-(2-(2-methoxyethoxy)ethoxy)ethyl)-benzamids

5,43 g (25 mmol) fein gemörstes $K_3PO_4$ wurde in einem Zweihalskolben mit Tropftrichter, Hahn und Magnetrührfisch in einem schwachen $N_2$-Strom mit einem Heizgebläse ausgeheizt. Unter $N_2$ wurde auf Raumtemperatur abgekühlt und anschließend 100 ml wasserfreies THF zugegeben. In einem Wägeschälchen wurden 1,84 g (10 mmol) 4-Dimethylaminobenzoylchlorid und 26 mg (0,07 mmol) Dibenzo-18-krone-6 abgewogen und zu dem Ansatz gegeben. Im Tropftrichter wurden 25 ml THF wasserfrei mit 1,63 g (10 mmol) 2-(2-(2-Methoxyethoxy)ethoxy)ethyl-amin vermischt. Unter $N_2$ wurde diese Lösung bei Raumtemperatur langsam zum Ansatz getropft. Nach beendeter Zugabe wurde ein $CaCl_2$-Trockenrohr auf den Ansatz gesetzt. Das Reaktionsgemisch wurde 24 h bei Raumtemperatur und anschließend 2,5 h bei 60 °C gerührt. Zur Aufarbeitung wurde der Ansatz über Celite 545 filtriert und der Filterrückstand mit 20 ml THF gewaschen. THF wurde am Rotationsverdampfer entfernt und der Rückstand in 100 Dichlormethan aufgenommen. Die organische Phase wurde mit 2x20 ml 0,5 N HCl-, 1x20 ml gesättigte $NaHCO_3$- und 1x20 ml gesättigte NaCl-Lösung extrahiert. Die organische Phase wurde über $Na_2SO_4$ getrocknet und das Lösungsmittel am Rotationsverdampfer entfernt. Die wässrigen Waschphasen wurden vereint und Wasser am Rotationsverdampfer entfernt. Der Rückstand wurde mit 100 ml Aceton und 50 ml Ethanol extrahiert. Das Aceton- und Ethanol-Extrakt wurde vereint und das Lösungsmittel am Rotationsverdampfer entfernt. Dünnschichtchromatograpie ($SiO_2$, Laufmittel Ethylacetat/Ethanol 95:5) der Rohprodukte aus der Dichlormethan-Phase und der wässrigen Phase zeigten beide einen Produktfleck bei $R_f$ = 0,22. Daraufhin wurden beide Rohprodukte in Dichlormethan gelöst, vereint und das Lösungsmittel am Rotationsverdampfer entfernt. Ausbeute Rohprodukt: 2,53 g. Weitere Aufreinigung erfolgte durch Mitteldruckchromatographie. Stationäre Phase: $SiO_2$. Laufmittel: Ethylacetat/Ethanol 95:5. Flussgeschwindigkeit: 40 ml/min. Säule: h = 46 cm, d = 2,6 cm. Ausbeute: 0,8 g.

$^1$H-NMR (CDCl3) ppm: 2,98 (s, 6h), 3,32 (s, 3H), 3,49-3,52 (m, 2H), 3,61-3,63 (m, 10H), 6,63 (d, 2H), 7,68 (d, 2H)

Synthese des 4-Dimethylamino-N-propylbenzamids

In einem Zweihalskolben mit Magnetrührfisch, Stopfen, Rückflusskühler und $CaCl_2$-Trockenrohr werden 1,66 g (10 mmol) 4-Dimethylaminobenzoesäure in 100 ml Dichlormethan suspendiert. Zu der Suspension werden 3,37 ml (20 mmol) Diisopropylethylamin gegeben, woraufhin eine klare Lösung entsteht. Der Lösung werden portionsweise 3,22 g (10 mmol) TBTU (2-(1H-Benzotriazol-1-yl)-tetramethyluronium tetrafluoroborat) zugefügt. Es entsteht eine leicht trübe, gelbe Lösung. Diese Mischung wird eine Stunde bei Raumtemperatur gerührt. Unter Kühlung werden dann 1,64 ml (20 mmol) n-Propylamin zugetropft. Der Ansatz erwärmt sich leicht dabei und die Farbe der Lösung hellt sich auf. Das Kühlwasser wird nach beendeter Zugabe ausgeschaltet und der Ansatz über Nacht bei Raumtemperatur gerührt. Es entsteht dabei eine leicht beige, klare Lösung.

Zur Aufarbeitung wird der Ansatz mit 20 ml einer 2N HCl-Lösung extrahiert. Anschließend wird mit 20 ml einer gesättigten $NaHCO_3$-Lösung extrahiert. Die organische Phase wird über $Na_2SO_4$ getrocknet, filtriert und das Lösungsmittel am Rotationsverdampfer entfernt. Als Rohprodukt fallen 3,04 g einer leicht grünlich-gelben, hochviskosen Flüssigkeit an. Das Rohprodukt wird durch Säulenchromatographie weiter aufgereinigt. Stationäre Phase: $Al_2O_3$, neutral. Laufmittel: Ethylacetat/n-Heptan 1:1. Säule: h = 50 cm, d = 4 cm. Das Produkt hat einen $R_f$ = 0,3. Die reinen Fraktionen werden gesammelt, das Lösungsmittel am Rotationsverdampfer entfernt und der Rückstand im Vakuum bei Raumtemperatur getrocknet. Das Produkt fällt als weißer Feststoff an. Ausbeute: 0,64 g. $^1$H-NMR ($CDCl_3$) ppm: 0,98 (t, 3H), 1,63 (sext., 2H), 3,02 (s, 6H), 3,42 (m, 2H), 6,03 (s, 1H), 6,72 (d, 2H), 7,69 (d, 2H)

Synthese des 3-Dimethylamino-N-propyl-benzamids

In einem Dreihalskolben mit Magnetrührfisch, Stopfen mit Innenthermometer, Tropftrichter, Rückflusskühler und $CaCl_2$-Trockenrohr werden 1,66 g (10 mmol) 3-Dimethylamino-benzoesäure in 50 ml Dichlormethan suspendiert. Zu der Suspension werden 3,37 ml (20 mmol) Diisopropylethylamin gegeben, woraufhin eine hellbraune, klare Lösung entsteht. Der Lösung werden portionsweise 3,21 g (10 mmol) TBTU (2-(1H-Benzotriazol-1-yl)-tetramethyluronium tetrafluoroborat) zugefügt. Es entsteht eine leicht trübe, gelbbraune Lösung. Diese Mischung wird eine Stunde bei Raumtemperatur gerührt. Im Tropftrichter wird eine Mischung aus 10 ml Dichlormethan und 1,64 ml (20 mmol) n-Propylamin zubereitet. Der Kolben wird mit einer Eis/Kochsalz-Mischung gekühlt. Bei ca. 0 °C Innentemperatur wird die Mischung aus dem Tropftrichter langsam innerhalb von 15 min zum Reaktionsgemisch getropft. 10 min nach Zugabe wird das Kältebad entfernt und der Ansatz 72 h bei Raumtemperatur gerührt. Zur Aufarbeitung wird dem Ansatz ca. 50 g $Al_2O_3$ neutral zugefügt und das Lösungsmittel am Rotationsverdampfer entfernt. Weitere Trocknung erfolgt im Vakuum bei Raumtemperatur. Das $Al_2O_3$ wird in eine Säule mit Fritte überführt und mit 800 ml einer Mischung aus Ethylacetat/n-Heptan 1:1 eluiert. Das Lösungsmittel wird am Rotationsverdampfer entfernt und der Rückstand im Vakuum bei Raumtemperatur getrocknet. Es fallen 3,31 g einer gelben, hochviskosen Flüssigkeit als Rohprodukt an. Durch Säulenchromatographie wird das Rohprodukt weiter aufgereinigt. Stationäre Phase: $SiO_2$. Laufmittel: Ethylacetat/n-Heptan 1:1. Säule: h = 50 cm, d = 3 cm. Das Produkt hat einen $R_f$ = 0,19. Die reinen Fraktionen werden gesammelt, das Lösungsmittel am Rotationsverdampfer entfernt und der Rückstand im Vakuum bei Raumtemperatur getrocknet. Das Produkt fällt als klare, leicht gelbliche, hochviskose Flüssigkeit an, die über Nacht im Kühlschrank zu einem leicht gelblichen Feststoff auskristallisiert. Ausbeute: 1,84 g. $^1$H-NMR (CDCl3) ppm: 0,98 (t, 3H), 1,63 (sext., 2H), 3,00 (s, 6H), 3,42 (m, 2H), 6,24 (s, 1H), 6,87 (dd, 1H), 7,00 (d, 1H), 7,24-7,30 (br, 2H)

Synthese des 4-Dimethylamino-N-(2-methoxyethyl)-benzamids

In einem Dreihalskolben mit Magnetrührfisch, Stopfen mit Innenthermometer, Tropftrichter, Rückflusskühler und $CaCl_2$-Trockenrohr werden 1,66 g (10 mmol) 4-Dimethylamino-benzoesäure in 90 ml Dichlormethan suspendiert. Zu der Suspension werden 3,37 ml (20 mmol) Diisopropylethylamin gegeben, woraufhin eine helle Lösung entsteht. Der Lösung werden portionsweise 3,22 g (10 mmol) TBTU (2-(1H-Benzotriazol-1-yl)-tetramethyluronium tetrafluoroborat) zugefügt. Es entsteht eine klare, gelbe Lösung. Diese Mischung wird eine Stunde bei Raumtemperatur gerührt. Im Tropftrichter wird eine Mischung aus 10 ml Dichlormethan und 1,72 ml (20 mmol) 2-Methoxyethylamin zubereitet. Der Kolben wird mit einer Eis/Kochsalz-Mischung gekühlt. Bei ca. 0 °C Innentemperatur wird die Mischung aus dem Tropftrichter langsam innerhalb von 15 min zum Reaktionsgemisch getropft. Es wird weitere 20 min gekühlt und dann das Kältebad entfernt. Eine klare Lösung mit einem weißen Feststoff ist entstanden. Bei Erwärmung auf Raumtemperatur löst sich der Feststoff wieder auf und die Lösung bleibt leicht trübe. Es wird 72 Stunden bei Raumtemperatur gerührt. Zur Aufarbeitung wird dem Ansatz ca. 40 g $Al_2O_3$ neutral zugefügt und das Lösungsmittel am Rotationsverdampfer entfernt. Weitere Trocknung erfolgt im Vakuum bei Raumtemperatur. Das $Al_2O_3$ wird in eine Säule mit Fritte überführt und mit einer Mischung aus Ethylacetat/n-Heptan 1:1 eluiert. Das Lösungsmittel wird am Rotationsverdampfer entfernt. Es

werden 2,90 g Rohprodukt isoliert. Weitere Aufreinigung erfolgt durch zweimaliges Umkristallisieren aus Ethylacetat. Das Produkt fällt als weißer Feststoff an. Ausbeute: 1,74 g. $^1$H-NMR ($CDCl_3$) ppm: 3,02 (s, 6H), 3,38 (s, 3H), 3,54 (t, 2H), 3,63 (m, 2H), 6,44 (s, 1H), 6,73 (d, 2H), 7,71 (m, 2H)

Synthese des 3-Dimethylamino-N-(2-methoxyethyl)-benzamids

In einem Dreihalskolben mit Magnetrührfisch, Innenthermometer, Rückflusskühler mit $CaCl_2$-Trockenrohr, sowie einem Tropftrichter mit Stopfen wurden 3,31 g (20mmol) 3-Dimethylaminobenzoesäure in 125 ml Dichlormethan suspendiert. Dazu wurden 6,62 ml (40 mmol) Ethyldiisopropylamin gegeben. Es entstand eine leicht bräunliche, klare Lösung. In einem Wägeschiffchen wurden 6,43 g (20 mmol) TBTU abgewogen und portionsweise bei Raumtemperatur zum Ansatz zugegeben. Es entstand eine gelbbraune Lösung. Die Lösung wurde 1 h bei Raumtemperatur gerührt. Anschließend wurde der Ansatz in einem Eisbad auf 0 °C gekühlt. Im Tropftrichter wurde eine Mischung aus 3,45 ml (40 mmol)2-Methoxyethylamin in 25 ml Dichlormethan zubereitet. Diese Lösung wurde innerhalb von 35 min zum Ansatz getropft, sodass die Innentemperatur nicht über 3 °C anstieg. Die Farbe des Reaktionsgemisches hellte sich auf und es entstand ein Feststoff. Nach beendeter Zugabe wurde das Eisbad entfernt und der Ansatz wurde 48 h bei Raumtemperatur gerührt. Zur Aufarbeitung wurde das Reaktionsgemisch in einen Scheidetrichter überführt und mit 2x50 ml 0,5 N HCl-, 1x50 ml gesättigter $NaHCO_3$- und 1x50 ml gesättigter NaCl-Lösung extrahiert. Die organsiche Phase wird über $Na_2SO_4$ getrocknet und das Lösungsmittel am Rotationsverdampfer entfernt. Ausbeute Rohprodukt: 2,85 g.

## Methoden

Alle Untersuchungen und Synthesen wurden bei 23±2°C und normalem Luftdruck durchgeführt, wenn nicht ausdrücklich etwas anderes angegeben ist.

Für die Herstellung von Pasten wurde ein Speedmixer DAC 150 FV der Firma Hauschild verwendet. Zur Homogenisierung der Pasten wurde ein Dreiwalzwerk Exakt 50 der Firma Exakt verwendet. Die Pasten wurden im Vakuum entgast.

Kleine Substanzmengen wurden mit dem Glasofen B 585 Kugelrohr der Firma Büchi destilliert.

Mitteldruckchromatographie

Es wurde ein MPLC-System der Firma Büchi verwendet. Es bestand aus einem Pumpenmodul C-601, einem Pumpen-Manager C-615 und einem Fraktionssammler C-660. Die zu chromatographierenden Proben wurden im Laufmittel gelöst und über ein 4-Wege-Injektionsventil auf eine Probenschlaufe (bis 20ml) aufgebracht. Es wurden Glassäulen vom Typ C-690 verwendet. Die genauen Dimensionen sind bei den Synthesen beschrieben.

$^1$H-NMR Spektroskopie

Die Messungen erfolgten auf einem Gemini 300 MHz NMR-Spektrometer der Firma Varian. Die Kalibrierung der Spektren erfolgte anhand des Restprotonen-Signals des verwendeten deuterierten Lösungsmittels.

Durchhärtetiefe (DHT)

Die Durchhärtetiefe wurde entsprechend der Prüfung "Polymerisationstiefe" nach der ISO 4049:2000 bestimmt. Hierzu wurden die Pasten in zylindrische Teflonformen (5 mm Durchmesser, 10 mm Höhe) gefüllt und mit einer Halogenlampe (Translux EC, Heraeus Kulzer) oder einer LED-Lampe (Mini LED, Acteon) 5, 10 oder 20 s von oben belichtet. Direkt nach der Belichtung wurden die ausgehärteten Prüfkörper entnommen und mit einem Messer von nicht ausgehärtetem Material befreit. Die Höhe des ausgehärteten Bereiches wurde mit einer Schieblehre ermittelt und als Durchhärtetiefe (DHT) in mm angegeben.

Biegefestigkeit

Mit Hilfe einer Form aus Kunststoff wurden Prüfkörper mit den Abmessungen (40±2) mm x (2±0,1) mm x (2±0,1) mm hergestellt. Hierzu wurden die jeweiligen Pasten im Lichtofen (HiLite Power, Heraeus Kulzer) lichtgehärtet. Die Pasten wurden in der Form zunächst 90 s belichtet, anschließend gewendet und nochmals 90 s belichtet. Nach Entformung wurden die Prüfkörper in VE-Wasser für 23 h bei 37 °C und dann 1 h bei 23 °C gelagert. Anschließend wurde mit Hilfe einer Universalprüfmaschine (Typ Z 010/TN2A, Zwick) die Biegefestigkeit (BF) gemäß ISO 4049:2000 gemessen. Die Messung erfolgte bei konstanter Vorschubgeschwindigkeit von 0,8 mm/min. Die Berechnung der BF [MPa] erfolgte nach der Formel:

$$BF = \frac{3 \times F \times l}{2 \times b \times h^2};$$

mit $F$ = maximale auf den Prüfkörper ausgeübte Kraft in Newton; $l$ = Abstand zwischen den Auflagen mit einer Genauigkeit von ± 0,01 mmm; b = Breite der Prüfkörper unmittelbar vor der Prüfung, gemessen in mm; h = Höhe der Prüfkörper unmittelbar vor der Prüfung gemessen in mm.

Scherhaftkraft (SBS) auf Rinderschmelz und Rinderdentin

Rinderzähne ohne Pulpen (Rocholl) wurden in ein kalt polymerisierendes Harz (Viscovoss GTS mit MEKP MEH-Härter, Voss Chemie) eingebettet. Unmittelbar vor der Versuchsdurchführung wurden pro Versuchsserie 10 Rinderzähne auf den gewünschten Untergrund (Schmelz oder Dentin) nass mit Schleifpapier 120 Grit abgeschliffen. Danach fand ein Feinschliff mit Schleifpapier der Körnung 500 Grit statt. Das Zahnmaterial hatte eine plane und glatte Oberfläche. Die geschliffenen Zähne wurden bis zur weiteren Behandlung in VE-Wasser aufbewahrt. In Etiketten wurden Löcher mit einem Durchmesser von 3 mm gestanzt. Der zu präparierende Zahn wurde unmittelbar vor der Behandlung aus dem Wasser genommen und die Feuchtigkeit mit ölfreier Druckluft entfernt, wobei ein übertrocknen vermieden wurde. Das gelochte Etikett wurde so mittig auf die Zahnoberfläche geklebt, dass das Loch für das Kleben zur Verfügung stand. Die Adhäsive wurden mit Hilfe eines Einmalpinsels 20 s auf der Zahnoberfläche verrieben und anschließend 20 s mit einer LED-Lampe (Mini LED, Acteon, blaues Licht, 182 $mW/cm^2$) belichtet. Anschließend wurde eine zweiteilige Teflonform (Höhe 3 mm) mit Bohrung (Durchmesser 3 mm) auf die präparierte Stelle plan aufgelegt und mit Metallklammern fixiert. Die Bohrung wurde mit dem Adhäsiv luftblasenfrei befüllt. Dann wurde 40 s mit einer LED-Lampe (Mini LED, Acteon) bestrahlt. Nach der Aushärtung wurde die Teflonform entfernt und überstehende Reste des ausgehärteten Adhäsivs mit einem Skalpell entfernt. Die so hergestellten Prüfkörper wurden danach 1 Tag bei 37 °C in einem Wasserbad gelagert. Unmittelbar vor der Messung der Scherhaftkraft ließ man das Wasserbad innerhalb einer Stunde auf 23 °C abkühlen.

Die Prüfkörper wurden in eine Abschervorrichtung gemäß ISO 10477:2004 zur Messung der Scherhaftkraft eingespannt und mit einer Universalprüfmaschine (Typ Z 010/TN2A, Zwick) bei einem Vorschub von 0,5 mm/min die SBS gemessen. Die Ergebnisse wurden in Form eines Mittelwerts mit Standardabweichung angegeben. Prüfkörper, deren Verklebung sich vor der Messung schon aufgelöst hat oder deren Verklebung nicht besser war als die eingestellte Vorkraft von 0,01 N, gehen mit einer Scherhaftkraft von 0 MPa in die Mittelwertbildung ein.

Bestimmung der Wasserlöslichkeit

Die Wasserlöslichkeit wurde in VE-Wasser durch UV/Vis-Spektroskopie bestimmt. Die Spektren wurden mit einem UV/Vis-Spektrometer (Evolution 600, Thermo Scientific) zwischen 200-900 nm aufgenommen. Die Messung erfolgte in Quarzküvetten (Schichtdicke 10 mm). Für das Hintergrundspektrum wurde das reine Lösungsmittel VE-Wasser verwendet. Von den Spektren der Coinitiatoren wurde das Hintergrundspektrum subtrahiert.

Verdünnte Lösungen der unterschiedlichen Coinitiatoren mit bekannter Konzentration wurden in VE-Wasser hergestellt. Die Absorption der unterschiedlichen Coinitiatoren wurde im Absorptionsmaximum (zwischen 290-310 nm) gemessen und eine lineare Beziehung zwischen der bekannten Konzentration und der gemessenen Absorption ermittelt.

Gesättigte, wässrige Lösungen der Coinitiatoren wurden soweit verdünnt, dass die gemessene Absorption in den oben gefundenen linearen Bereich fällt. Über das Lambert-Beer-Gesetz wird die Konzentration der Coinitiatoren in der gesättigten, wässrigen Lösung ermittelt.

Folgende Löslichkeiten in Wasser wurden ermittelt:

| | **Coinitiator** | **Löslichkeit [mg/l]** |
|---|---|---|
| | 4-Dimethylaminobenzoesäure-ethylester[1)] | 36 |
| | 4-Dimethylaminobenzoesäure-2-n-butoxyethylester[1)] | 35 |
| EHA | 4-Dimethylaminobenzoesäure-2-ethylhexylester[1)] | 26 |
| | 4-Dimethylaminobenzonitril[1)] | 278 |
| PEGA | 4-Dimethylaminobenzoesäure-poly(ethylenglykol)monomethylether-ester | >766300 |
| | 4-Dimethylaminobenzamid | 427 |
| | 4-Dimethylamino-N-propyl-benzamid | 680 |

(fortgesetzt)

| | Coinitiator | Löslichkeit [mg/l] |
|---|---|---|
| | 4-Dimethylamino-N-(2-methoxyethyl)-benzamid | 2670 |
| [1)] Coinitiator gemäß Stand der Technik | | |

**Beispielzusammensetzungen**

**Beispiel 1**

Es wurden einteilige radikalisch polymerisierbare lichthärtbare adhäsive Dentalkomposite hergestellt und Ihre Haftkraft (SBS), Durchhärtungstiefe (DHT) und Biegefestigkeit (BF) gemessen.

Zunächst wurden aus den in den Tabellen jeweils angegebenen Komponenten Harzzusammensetzungen hergestellt. Diese Harzzusammensetzungen dienten als Matrix für die zur Pastenherstellung zugefügten Füllstoffe. Zur Herstellung der Kompositpasten wurden in 37 Teilen Harzmatrix 60 Teile Glaspulver und 3 Teile Kieselsäure suspendiert. Die Herstellung erfolgte ansonsten wie bei den Methoden beschrieben.

| | **Vergleich 1** | **Vergleich 2** | **Vergleich 3** | **Erfindungsgemäß** | **Erfindungsgemäß** |
|---|---|---|---|---|---|
| | **Composite 3** | **Composite 11** | **Composite 4** | **Composite 9** | **Composite 6** |
| Harz 55/45* | **64,15** | **64,38** | **79,1** | **64,04** | **78,7** |
| HEMA | **24,95** | **24,76** | **11,99** | **24,63** | **11,93** |
| MPD | **9,98** | **9,91** | **8,04** | **9,86** | **7,95** |
| BHT | **0,05** | **0,05** | **-** | **0,05** | **-** |
| CQ | **0,2** | **0,2** | **0,2** | **0,2** | **0,2** |
| EHA | **0,67** | **0,7** | **0,67** | **-** | **-** |
| PEGA | **-** | **-** | **-** | **1,22** | **1,22** |
| | | | | | |
| BF | **131** | **-** | **148** | **-** | **136** |
| DHT (5s) | **7,7** | **-** | **9,1** | **-** | **8,7** |
| DHT (10 s) | **9,1** | **-** | **9,9** | **-** | **9,8** |
| DHT (20s) | **10** | **-** | **-** | **-** | **-** |
| SBS Schmelz (SD) | **-** | **5,95 (2,15)** | **-** | **5,12 (1,5)** | **-** |
| SBS Dentin (SD) | - | **2,34 (1,4)** | - | **5 (2,2)** | - |
| * (Bis-GMA 54,92; TEGDMA 44,93; BHT 0,15%) | | | | | |

Für den Photoinitiator CQ erkennt man, dass beim äquimolaren Austausch eines Coinitiators des Standes der Technik (EHA, Composite 11) durch einen erfindungsgemäßen Coinitiator (PEGA, Composite 9), der eine höherer Wasserlöslichkeit aufweist, nahezu eine Verdopplung der Haftkraft auf Dentin erzielt wird. Gleichzeitig werden gute Werte für die DHT und BF erreicht.

**Beispiel 2**

| | Vergleich 4 | Vergleich 5 | Vergleich 6 | Erfindungsgemäß |
|---|---|---|---|---|
| | Composite 1 | Composite 2 | Composite 12 | Composite 10 |
| Harz 55/45* | 64,08 | 64,06 | 64,28 | 63,93 |
| HEMA | 24,93 | 24,92 | 24,73 | 24,59 |
| MPD | 9,97 | 9,97 | 9,89 | 9,84 |
| BHT | 0,05 | 0,05 | 0,05 | 0,05 |
| CQ-10-sulfonsäure | 0,3 | - | - | - |
| CQ-10-sulfonat | - | 0,33 | 0,36 | 0,36 |
| EHA | 0,67 | 0,67 | 0,69 | - |
| PEGA | - | - | - | 1,22 |
| | | | | |
| BF | 93 | 102 | - | - |
| DHT (5s) | 3,8 | 4,4 | - | - |
| DHT (10 s) | 6,3 | 7,2 | - | - |
| DHT (20s) | 8,7 | 9 | - | - |
| SBS Schmelz (SD) | - | - | 3,3 (1,45) | 4,13 (1,56) |
| SBS Dentin (SD) | - | - | 1 (1,66) | 4,68 (2,23) |
| * (Bis-GMA 54,92; TEGDMA 44,93; BHT 0,15%) | | | | |

Bei äquimolarem Austausch des Photoinitiators CQ durch seine hydrophileren Derivate wurde überraschend gefunden, dass auch mit diesen durch einen erfindungsgemäßen Coinitiator mit höherer Wasserlöslichkeit (PEGA, Composite 10) eine sprunghafte Verbesserung der Haftkraft auf Dentin erzielt wird, jedoch sind die absoluten Werte nicht weiter verbessert. Die Kombination von CQ-10-sulfonat und EHA führt andererseits zu einer signifikanten Verschlechterung der Haftung.

Die Kombination aus hydrophilem Photoinitiator und hydrophobem Coinitiator (C12) hatte die geringsten Scherhaftkräfte, sowohl auf Schmelz als auch auf Dentin. Die Kombination aus hydrophobem Photoinitiator und hydrophilem Coinitiator (C9) lieferte auf Schmelz eine vergleichbare Scherhaftkraft wie C11. Die Scherhaftung auf Dentin war dagegen signifikant höher als bei C11. Die Kombination aus hydrophilem Photoinitiator und hydrophilem Coinitiator (C10) lieferte im Vergleich zu C9 und C11 eine etwas geringere Scherhaftkraft auf Schmelz. Die Scherhaftkraft auf Dentin war dagegen ebenfalls signifikant höher als bei C11. Aus dieser Testreihe ist ersichtlich, dass ein hydrophiles bzw. wasserlösliches, aromatisches, tertiäres Amin als Coinitiator eine deutliche Verbesserung der Haftung auf Dentin ermöglicht.

**Beispiel 3**

| | Erfindungsgemäß | Erfindungsgemäß | Erfindungsgemäß | Erfindungsgemäß |
|---|---|---|---|---|
| | Composite 5 | Composite 6 | Composite 7 | Composite 8 |
| Harz 55/45* | 79,17 | 78,7 | 78,21 | 77,73 |
| HEMA | 12,01 | 11,93 | 11,86 | 11,79 |
| MPD | 8 | 7,95 | 7,9 | 7,85 |
| CQ | 0,2 | 0,2 | 0,2 | 0,2 |
| PEGA | 0,62 | 1,22 | 1,83 | 2,43 |
| | | | | |

(fortgesetzt)

| | Erfindungsgemäß | Erfindungsgemäß | Erfindungsgemäß | Erfindungsgemäß |
|---|---|---|---|---|
| | Composite 5 | Composite 6 | Composite 7 | Composite 8 |
| BF | 129 | 136 | 140 | 145 |
| DHT (5 s) | 8,2 | 8,7 | 9 | 9 |
| DHT (10 s) | 9,7 | 9,8 | 9,9 | 9,9 |
| * (Bis-GMA 54,92; TEGDMA 44,93; BHT 0,15%) | | | | |

Durch Erhöhung des Anteils an erfindungsgemäßem Coinitiator in den erfindungsgemäßen Kompositen lässt sich insbesondere die Biegefestigkeit weiter verbessern.

Das molare Verhältnis von Photoinitiator zu Coinitiator ist in folgender Tabelle aufgeführt:

| | Composites | | | | |
|---|---|---|---|---|---|
| | 4 | 5 | 6 | 7 | 8 |
| molares Verhältnis Photoinitiator zu Coinitiator | 1:2,03 | 1:1,04 | 1:2,04 | 1:3,06 | 1:4,07 |

## Patentansprüche

**1.** Dentalmaterial, das enthält:

a) wenigstens ein Monomer enthaltend mindestens eine radikalisch polymerisierbare ethylenisch ungesättigte Gruppe und mindestens eine Säuregruppe,
b) wenigstens ein Monomer, das keine Säuregruppen und mindestens eine radikalisch polymerisierbare ethylenisch ungesättigte Gruppe enthält,
c) wenigstens einen Photoinitiator,
d) mindestens ein tertiäres aromatisches Amin als Coinitiator, das einen Benzolring aufweist, an den mindestens eine Dialkylamingruppe und mindestens eine weitere Gruppe direkt gebunden sind,

**dadurch gekennzeichnet, dass** die weitere Gruppe ausgewählt ist aus:

i. Carbonsäureestergruppen enthaltend mindestens eine Polyoxyalkylengruppe mit mindestens 2 Oxyethylen- und/oder Oxypropyleneinheiten, und
ii. Amidgruppen.

**2.** Dentalmaterial nach Anspruch 1, **dadurch gekennzeichnet, dass** die mindestens eine weitere Gruppe mindestens eine Polyoxyalkylengruppe mit 3 bis 20, vorzugsweise 5 bis 17 Oxyethylen- und/oder Oxypropyleneinheiten enthält.

**3.** Dentalmaterial nach Anspruch 1 oder 2, **dadurch gekennzeichnet, dass** das tertiäre aromatische Amin ausgewählt ist aus der Gruppe bestehend aus:

a)

Formel I

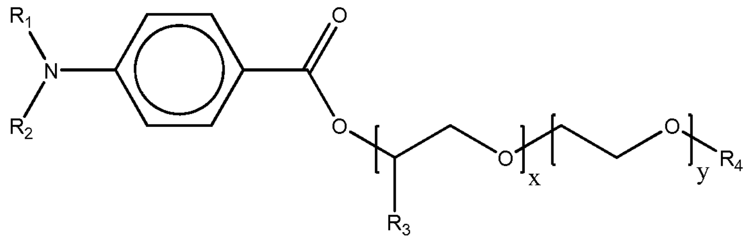

b)

Formel II

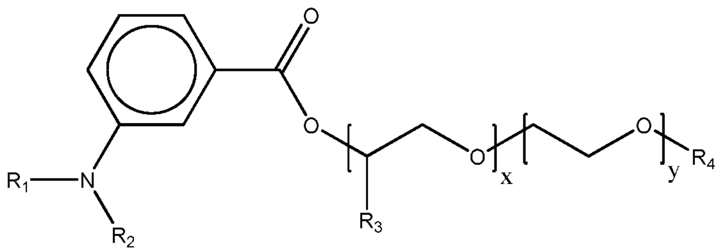

c)

Formel III

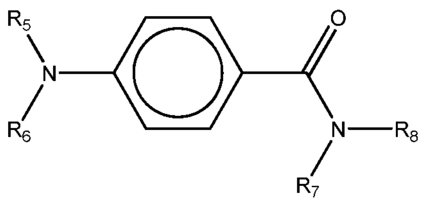

d)

Formel IV

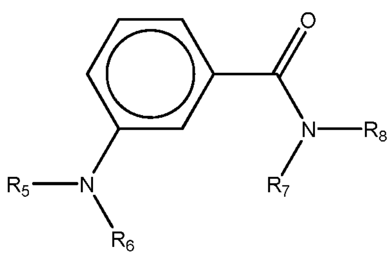

wobei in den Formeln bedeuten:

- $R_1$ und $R_2$ sind unabhängig voneinander) C1-C5-Alkyl, bevorzugt Methyl,

- $R_3$ ist Alkyl, bevorzugt Methyl,
- $R_4$ ist H oder C1-C5-Alkyl, bevorzugt Methyl,
- $R_5$ und $R_6$ sind unabhängig voneinander C1-C5-Alkyl, bevorzugt Methyl,
- $R_7$ und $R_8$ sind unabhängig voneinander ausgewählt aus H, Alkyl, Alkoxyalkyl und Polyalkylenoxid, bevorzugt Polyalkylenoxid mit mindestens zwei Oxyethylen- und/oder Oxypropyleneinheiten,

$x \geq 0$ und $y > 1$.

4. Dentalmaterial nach Anspruch 3, **dadurch gekennzeichnet, dass** die Summe aus x und y 3 bis 20, vorzugsweise 5 bis 17 beträgt.

5. Dentalmaterial nach Anspruch 3 oder 4, **dadurch gekennzeichnet, dass** x kleiner als y ist.

6. Dentalmaterial nach einem der Ansprüche 1 bis 5, **dadurch gekennzeichnet, dass** die Wasserlöslichkeit eines Coinitiators enthaltend wenigstens eine Amidgruppe wenigstens 0,4 g/l, vorzugsweise wenigstens 2 g/l, weiter vorzugsweise wenigstens 50 g/l beträgt; und dass die Wasserlöslichkeit eines Co-Initiators enthaltend keine Amidgruppe wenigstens 2 g/l, weiter vorzugsweise wenigstens 50 g/l beträgt.

7. Dentalmaterial nach einem der Ansprüche 1 bis 5, **dadurch gekennzeichnet, dass** der Gehalt des Co-Initiators 0,1 bis 10 Gew.-%, vorzugsweise 0,5 bis 5 Gew.-% beträgt.

8. Dentalmaterial nach einem der Ansprüche 1 bis 7, **dadurch gekennzeichnet, dass** das wenigstens eine Monomer, das keine Säuregruppen und mindestens eine radikalisch polymerisierbare ethylenisch ungesättigte Gruppe enthält, ausgewählt ist aus der Gruppe bestehend aus:

b1)

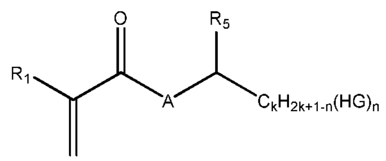

wobei in der Formel bedeutet:

$R^1$ ist H, Methyl oder Ethyl, bevorzugt H oder Methyl;
A ist O oder $NR^3$, mit $R^3$ = H, Methyl, Ethyl oder Propyl, bevorzugt $R^3$ = H;
HG ist eine hydrophile Gruppe ausgewählt aus OH und $(O-CH_2-CH_2)_m-OR^4$; bevorzugt ist HG = OH;
$R^5$ ist H, Methyl, Ethyl oder $C_kH_{2k+1-n}(HG)_n$; bevorzugt H;
$R^4$ ist H oder Methyl;
k, m, n sind natürliche Zahlen;
k = 1-5; bevorzugt 1-2;
m= 3-20; bevorzugt 5-20;
n= 1-5;
$n \leq k$;
jedes Kohlenstoffatom trägt höchstens eine hydrophile Gruppe HG;

b2) $(Q\ A\ E_v)_rC_tH_{2t+2-(r+s)}(HG)_s$
mit Q =

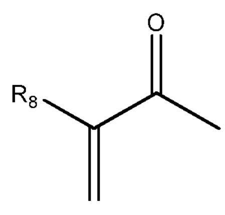

E =

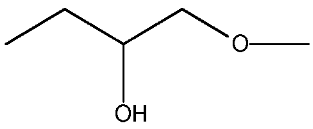

oder

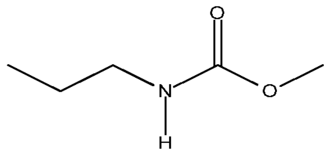

wobei in den Formeln bedeuten:

$R^8$ ist H, Methyl oder Ethyl, bevorzugt H oder Methyl;
A ist O oder $NR^3$, mit $R^3$ = H, Methyl, Ethyl oder Propyl, bevorzugt $R^3$ = H;
HG ist OH, $(O\text{-}CH_2\text{-}CH_2)_m\text{-}OR^7$; bevorzugt OH;
$R^7$ ist H oder Methyl;
m, r, s, t, v sind natürliche Zahlen;
m= 3-20, bevorzugt 6-20;
r= 2-4;
s= 1-4;
t= 2-8; bevorzugt 2-6;
v= 0 oder 1; bevorzugt 0; und

b3) $Q\text{-}A\text{-}E_v\text{-}C_2H_2\text{-}HG\text{-}E_v\text{-}A\text{-}Q$
mit Q =

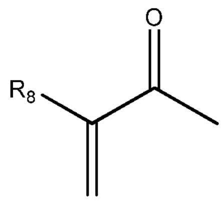

E =

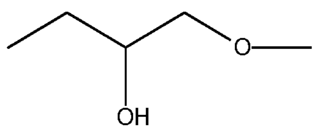

oder

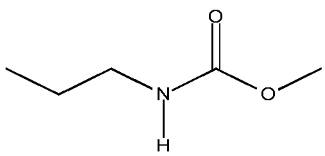

wobei in den Formeln bedeuten:

$R^8$ ist H, Methyl oder Ethyl, bevorzugt H oder Methyl;
A ist O oder $NR^3$, mit $R^3$ = H, Methyl, Ethyl oder Propyl, bevorzugt $R^3$ = H;
HG ist $(O\text{-}CH_2\text{-}CH_2)_m$;
m= 4-20, bevorzugt 6-20;
v= 0 oder 1; bevorzugt 0.

**9.** Dentalmaterial nach einem der Ansprüche 1 bis 8, **dadurch gekennzeichnet, dass** es zusätzlich wenigstens ein Monomer b4), das keine Säuregruppen und mindestens zwei radikalisch polymerisierbare ethylenisch ungesättigte Gruppen enthält, aufweist, wobei dieses wenigstens eine Monomer b4) nicht aus der Gruppe b1), b2) und b3) ausgewählt ist.

**10.** Dentalmaterial nach einem der Ansprüche 8 bis 9, **dadurch gekennzeichnet, dass** das wenigstens eine Monomer b1) bis b3) eine Wasserlöslichkeit von wenigstens 50 g/l aufweist.

**11.** Dentalmaterial nach Anspruch 9 oder 10, **dadurch gekennzeichnet, dass** das wenigstens eine Monomer b4)) eine Wasserlöslichkeit von weniger als 50 g/l aufweist.

**12.** Dentalmaterial nach einem der Ansprüche 1 bis 11 zur Verwendung als lichthärtendes selbsthaftendes und selbstätzendes radikalisch polymerisierbares Dentalrestaurativ.

**13.** Verwendung eines tertiären aromatisches Amins, das einen Benzolring aufweist, an den mindestens eine Dialkylamingruppe und mindestens eine weitere Gruppe direkt gebunden sind, wobei die weitere Gruppe ausgewählt ist aus:

i. Carbonsäureestergruppen enthaltend mindestens eine Polyoxyalkylengruppe mit mindestens 2 Oxyethylen- und/oder Oxypropyleneinheiten, und
ii. Amidgruppen

als Coinitiator in einem polymerisierbaren Dentalmaterial.

## Claims

**1.** Dental material, comprising:

a) at least one monomer comprising at least one radically polymerizable ethylenically unsaturated group and at least one acid group,
b) at least one monomer not comprising any acid groups and comprising at least one radically polymerizable ethylenically unsaturated group,
c) at least one photoinitiator,
d) at least one tertiary aromatic amine as coinitiator, which amine exhibits a benzene ring to which at least one dialkylamine group and at least one additional group are directly bonded,

**characterized in that** the additional group is chosen from:

i. carboxylic acid ester groups comprising at least one polyoxyalkylene group with at least 2 oxyethylene and/or oxypropylene units, and
ii. amide groups.

**2.** Dental material according to Claim 1, **characterized in that** the at least one additional group comprises at least one polyoxyalkylene group with from 3 to 20, preferably from 5 to 17, oxyethylene and/or oxypropylene units.

**3.** Dental material according to Claim 1 or 2, **characterized in that** the tertiary aromatic amine is chosen from the group consisting of:

a)

formula I

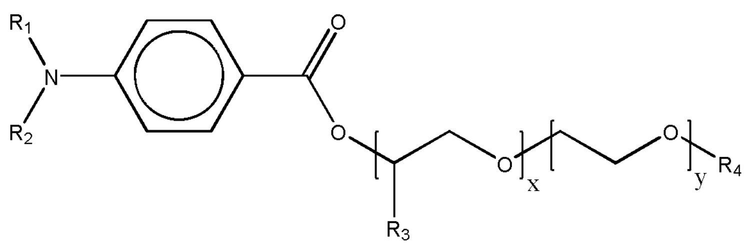

b)

formula II

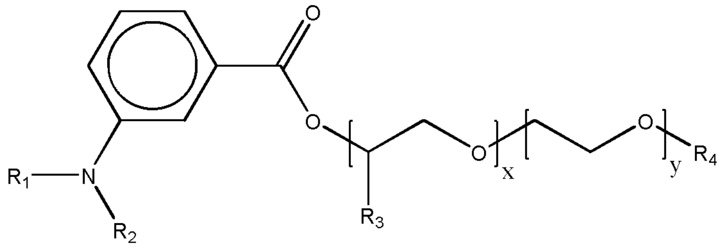

c)

formula III

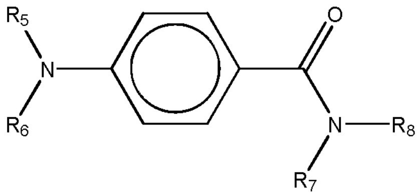

d)

formula IV

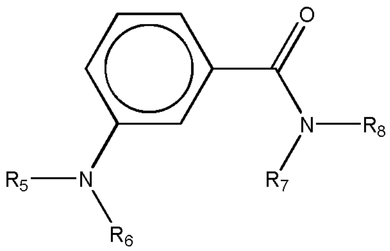

in which, in the formulae represented:

- $R_1$ and $R_2$ are, independently of one another, $C_1$-$C_5$-alkyl, preferably methyl,
- $R_3$ is alkyl, preferably methyl,
- $R_4$ is H or $C_1$-$C_5$-alkyl, preferably methyl,
- $R_5$ and $R_6$ are, independently of one another, $C_1$-$C_5$-alkyl, preferably methyl,
- $R_7$ and $R_8$ are, independently of one another, chosen from H, alkyl, alkoxyalkyl and polyalkylene oxide, preferably polyalkylene oxide with at least two oxyethylene and/or oxypropylene units,

$x \geq 0$ and $y > 1$.

4. Dental material according to Claim 3, **characterized in that** the sum of x and y amounts to from 3 to 20, preferably from 5 to 17.

5. Dental material according to Claim 3 or 4, **characterized in that** x is smaller than y.

6. Dental material according to one of Claims 1 to 5, **characterized in that** the water solubility of a coinitiator comprising at least one amide group is at least 0.4 g/l, preferably at least 2 g/l, more preferably at least 50 g/l; and **in that** the water solubility of a coinitiator not comprising any amide group is at least 2 g/l, more preferably at least 50 g/l.

7. Dental material according to one of Claims 1 to 5, **characterized in that** the content of the coinitiator is from 0.1% to 10% by weight, preferably from 0.5% to 5% by weight.

8. Dental material according to one of Claims 1 to 7, **characterized in that** the at least one monomer not comprising any acid groups and comprising at least one radically polymerizable ethylenically unsaturated group is chosen from the group consisting of:

b1)

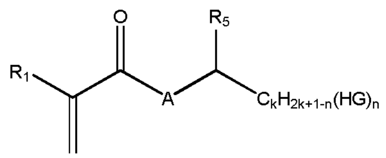

in which, in the formula represented:

$R_1$ is H, methyl or ethyl, preferably H or methyl;
A is O or $NR_3$, with $R_3$ = H, methyl, ethyl or propyl, preferably $R_3$ = H;
HG is a hydrophilic group chosen from OH and $(O-CH_2-CH_2)_m-OR_4$; preferably HG = OH;
$R_5$ is H, methyl, ethyl or $C_kH_{2k+1-n}(HG)_n$; preferably H;
$R_4$ is H or methyl;
k, m, n are natural numbers;
k = 1-5; preferably 1-2;
m = 3-20; preferably 5-20;
n = 1-5;
$n \leq k$;
each carbon atom carries at most one hydrophilic group HG;

b2) $(QAE_v)rC_tH_{2t+2-(r+s)}(HG)_s$
with Q =

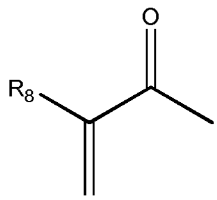

E =

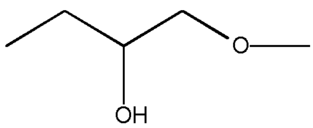

or

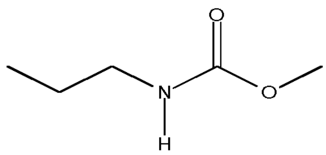

in which, in the formulae represented:

$R_8$ is H, methyl or ethyl, preferably H or methyl;
A is O or $NR_3$, with $R_3$ = H, methyl, ethyl or propyl, preferably $R_3$ = H;
HG is OH or $(O-CH_2-CH_2)_m-OR_7$; preferably OH;
$R_7$ is H or methyl;
m, r, s, t, v are natural numbers;
m = 3-20, preferably 6-20;
r = 2-4;
s = 1-4;

t = 2-8; preferably 2-6;
v = 0 or 1; preferably 0; and

b3) $Q\text{-}A\text{-}E_v\text{-}C_2H_2\text{-}HG\text{-}E_v\text{-}A\text{-}Q$
with Q =

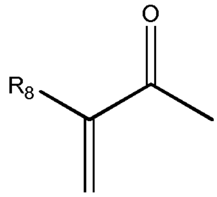

E =

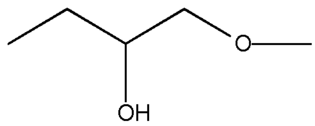

or

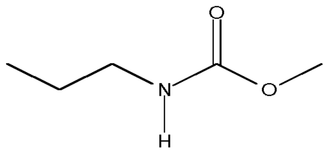

in which, in the formulae represented:

$R_8$ is H, methyl or ethyl, preferably H or methyl;
A is O or $NR_3$, with $R_3$ = H, methyl, ethyl or propyl, preferably $R_3$ = H;
HG is $(O\text{-}CH_2\text{-}CH_2)_m$;
m = 4-20, preferably 6-20;
v = 0 or 1; preferably 0.

**9.** Dental material according to one of Claims 1 to 8, **characterized in that** it additionally exhibits at least one monomer b4) not comprising any acid groups and comprising at least two radically polymerizable ethylenically unsaturated groups, this at least one monomer b4) not being chosen from the group b1), b2) and b3) .

**10.** Dental material according to either of Claims 8 and 9, **characterized in that** the at least one monomer b1) to b3) exhibits a water solubility of at least 50 g/l.

**11.** Dental material according to Claim 9 or 10, **characterized in that** the at least one monomer b4), exhibits a water solubility of less than 50 g/l.

**12.** Dental material according to one of Claims 1 to 11, for use as light-curing, self-bonding and self-etching radically polymerizable dental restoration material.

**13.** Use of a tertiary aromatic amine which exhibits a benzene ring to which at least one dialkylamine group and at least one additional group are directly bonded, the additional group being chosen from:

i. carboxylic acid ester groups comprising at least one polyoxyalkylene group with at least 2 oxyethylene and/or oxypropylene units, and
ii. amide groups, as coinitiator in a polymerizable dental material.

## Revendications

**1.** Matériau dentaire, qui contient :

a) au moins un monomère contenant au moins un groupe éthyléniquement insaturé polymérisable par voie radicalaire et au moins un groupe acide,
b) au moins un monomère, qui ne contient pas de groupe acide et qui contient au moins un groupe éthyléniquement insaturé polymérisable par voie radicalaire,
c) au moins un photo-initiateur,
d) au moins une amine aromatique tertiaire en tant que co-initiateur, qui présente un cycle aromatique, auquel sont directement liés au moins un groupe dialkylamine et au moins un autre groupe,

**caractérisé en ce que** l'autre groupe est choisi parmi :

i. des groupes ester carboxylique contenant au moins un groupe poly(oxyde d'alkylène) comportant au moins 2 unités oxyde d'éthylène et/ou oxyde de propylène, et
ii. des groupes amide.

**2.** Matériau dentaire selon la revendication 1, **caractérisé en ce que** l'au moins un autre groupe contient au moins un groupe poly(oxyde d'alkylène) comportant 3 à 20, de préférence 5 à 17 unités oxyde d'éthylène et/ou oxyde de propylène.

**3.** Matériau dentaire selon la revendication 1 ou 2, **caractérisé en ce que** l'amine aromatique tertiaire est choisie dans le groupe constitué par :

a)

Formule I

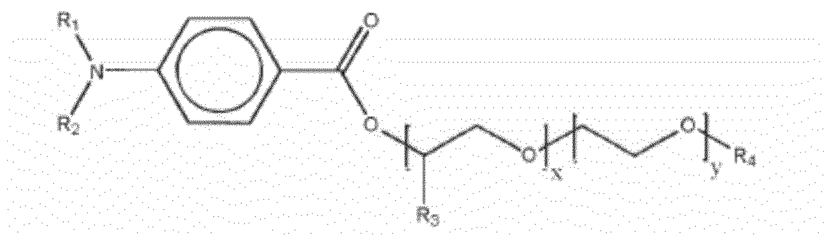

b)

Formule II

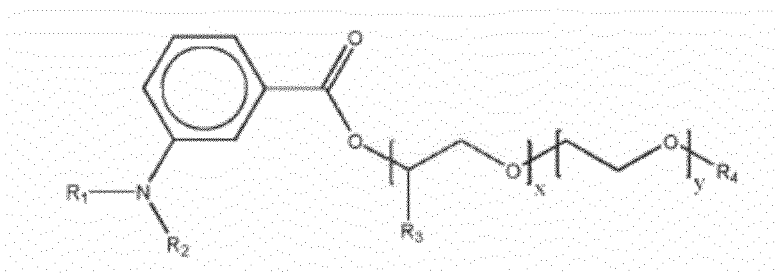

c)

Formule III

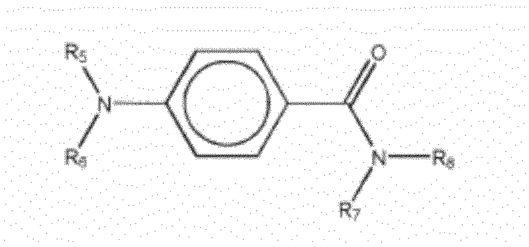

d)

Formule IV

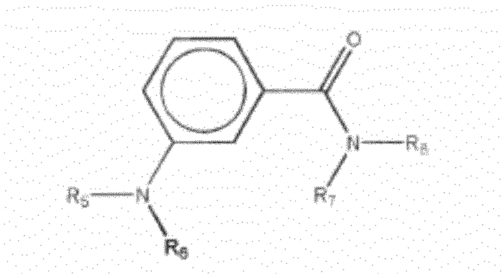

où dans les formules on a les significations :

- $R_1$ et $R_2$ sont indépendamment l'un de l'autre $C_{1-5}$-alkyle, préférablement méthyle,
- $R_3$ est alkyle, préférablement méthyle,
- $R_4$ est H ou $C_{1-5}$-alkyle, préférablement méthyle,
- $R_5$ et $R_6$ sont indépendamment l'un de l'autre $C_{1-5}$-alkyle, préférablement méthyle,
- $R_7$ et $R_8$ sont choisis indépendamment l'un de l'autre parmi H, alkyle, alcoxyalkyle et poly(oxyde d'alkylène), préférablement poly(oxyde d'alkylène) comportant au moins deux unités oxyde d'éthylène et/ou oxyde de propylène,

$x \geq 0$ et $y \geq 1$.

**4.** Matériau dentaire selon la revendication 3, **caractérisé en ce que** la somme de x et y est de 3 à 20, de préférence de 5 à 17.

**5.** Matériau dentaire selon la revendication 3 ou 4, **caractérisé en ce que** x est inférieur à y.

**6.** Matériau dentaire selon l'une quelconque des revendications 1 à 5, **caractérisé en ce que** la solubilité dans l'eau d'un co-initiateur contenant au moins un groupe amide est d'au moins 0,4 g/l, préférablement d'au moins 2 g/l, plus préférablement d'au moins 50 g/l ; et la solubilité dans l'eau d'un co-initiateur ne contenant pas de groupe amide est d'au moins 2 g/l, plus préférablement d'au moins 50 g/l.

**7.** Matériau dentaire selon l'une quelconque des revendications 1 à 5, **caractérisé en ce que** la teneur en co-initiateur est de 0,1 à 10% en poids, préférablement de 0,5 à 5% en poids.

**8.** Matériau dentaire selon l'une quelconque des revendications 1 à 7, **caractérisé en ce que** l'au moins un monomère, qui ne contient pas de groupes acide et qui contient au moins un groupe éthyléniquement insaturé polymérisable par voie radicalaire, est choisi dans le groupe constitué par :

b1)

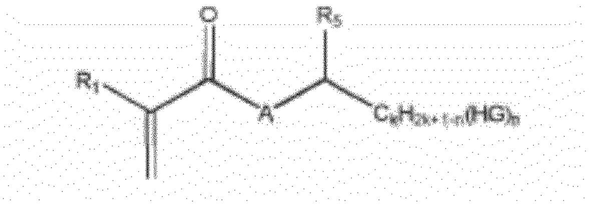

où dans la formule on a les significations :

- $R^1$ est H, méthyle ou éthyle, préférablement H ou méthyle ;
- A est O ou $NR_3$, avec $R^3$ = H, méthyle, éthyle ou propyle, préférablement $R^3$ = H ;
- HG est un groupe hydrophile choisi parmi OH et $(O\text{-}CH_2\text{-}CH_2)_m\text{-}OR^4$ ; préférablement HG = OH ;
- $R^5$ est H, méthyle, éthyle ou $C_kH_{2k+1-n}(HG)_n$ ; préférablement H ;
- $R^4$ est H ou méthyle ;
- k, m, n sont des entiers naturels ;
- k = 1 à 5 ; préférablement 1 à 2 ;
- m = 3 à 20 ; préférablement 5 à 20 ;
- n = 1 à 5 ;
- $n \leq k$ ;
- chaque atome de carbone porte au plus un groupe hydrophile HG ;

b2) $(Q\ A\ E_v)_rC_tH_{2t+2-(r+s)}(HG)_s$
avec Q =

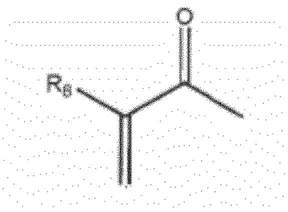

E =

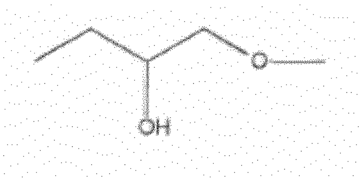

ou

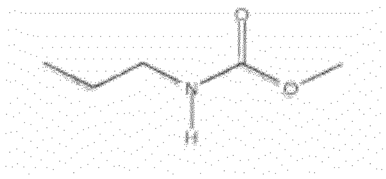

où dans les formules on a les significations :

- $R^8$ est H, méthyle ou éthyle, préférablement H ou méthyle ;
- A est O ou $NR_3$, avec $R^3$ = H, méthyle, éthyle ou propyle, préférablement $R^3$ = H ;
- HG est OH, $(O\text{-}CH_2\text{-}CH_2)_m\text{-}OR^7$ ; préférablement OH ;
- $R^7$ est H ou méthyle ;
- m, r, s, t, v sont des entiers naturels ;
- m = 3 à 20, préférablement 6 à 20 ;
- r = 2 à 4 ;
- S = 1 à 4 ;
- t = 2 à 8 ; préférablement 2 à 6 ;
- v = 0 ou 1 ; préférablement 0 ; et

b3) $Q\text{-}A\text{-}E_v\text{-}C_2H_2\text{-}HG\text{-}E_v\text{-}A\text{-}Q$
avec Q =

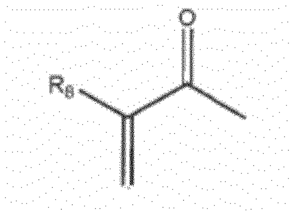

E =

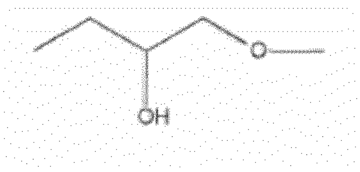 ou 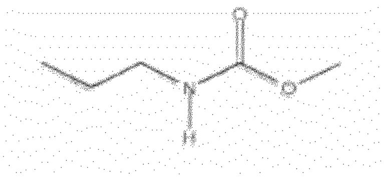

où dans les formules on a les significations :

$R^8$ est H, méthyle ou éthyle, préférablement H ou méthyle ;
A est O ou $NR_3$, avec $R^3$ = H, méthyle, éthyle ou propyle, préférablement $R^3$ = H ;
HG est $(O\text{-}CH_2\text{-}CH_2)_m$ ;
m = 4 à 20, préférablement 6 à 20 ;
v = 0 ou 1 ; préférablement 0.

**9.** Matériau dentaire selon l'une quelconque des revendications 1 à 8, **caractérisé en ce qu'**il présente de plus au moins un monomère b4), qui ne contient pas de groupes acide et qui contient au moins deux groupes éthyléniquement insaturés polymérisables par voie radicalaire, cet au moins un monomère b4) n'étant pas choisi dans le groupe de b1), b2) et b3).

**10.** Matériau dentaire selon l'une quelconque des revendications 8 à 9, **caractérisé en ce que** l'au moins un monomère b1) à b3) présente une solubilité dans l'eau d'au moins 50 g/l.

**11.** Matériau dentaire selon la revendication 9 ou 10, **caractérisé en ce que** l'au moins un monomère b4) présente une solubilité dans l'eau inférieure à 50 g/l.

**12.** Matériau dentaire selon l'une quelconque des revendications 1 à 11 pour une utilisation en tant que restauration dentaire polymérisable par voie radicalaire durcissant à la lumière, auto-adhésive et auto-mordançante.

**13.** Utilisation d'une amine aromatique tertiaire, qui présente un cycle aromatique, auquel sont directement liés au moins un groupe dialkylamine et au moins un autre groupe, l'autre groupe étant choisi parmi :

i. des groupes ester carboxylique contenant au moins un groupe poly(oxyde d'alkylène) comportant au moins 2 unités oxyde d'éthylène et/ou oxyde de propylène, et
ii. des groupes amide

en tant que co-initiateur dans un matériau dentaire polymérisable.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2011139936 A
- EP 2286784 A
- EP 2153811 A
- WO 2007100569 A
- EP 1784155 A
- EP 1387657 A
- EP 0136186 A
- US 20110275035 A1
- WO 0049064 A
- US 6620846 B2
- EP 2133368 A, Sekiguchi
- EP 1720506 A, Neffgen